(19)

Europäisches
Patentamt
European
Patent Office
Office européen
des brevets

(11)     EP 3 172 565 B1

(12)     **EUROPEAN PATENT SPECIFICATION**

(45) Date of publication and mention
     of the grant of the patent:
     **10.04.2019 Bulletin 2019/15**

(51) Int Cl.:
     **G01N 33/552** (2006.01)

(21) Application number: **15744286.4**

(86) International application number:
     **PCT/GB2015/052107**

(22) Date of filing: **22.07.2015**

(87) International publication number:
     **WO 2016/012778 (28.01.2016 Gazette 2016/04)**

(54) **CAPILLARY ASSAY DEVICE WITH INTERNAL HYDROPHYLIC COATING**

  KAPILLARENASSAYVORRICHTUNG MIT HYDROPHILER DECKSCHICHT

  DISPOSITIF D'ESSAI A CAPILLAIRES À CHOUCHE DE REVÊTEMENT HYDROPHILE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.07.2014 GB 201413229**

(43) Date of publication of application:
**31.05.2017 Bulletin 2017/22**

(73) Proprietor: **Loughborough University
Leicestershire LE11 3TU (GB)**

(72) Inventors:
  • **EDWARDS, Alexander Daniel
    Reading
    Berkshire RG6 6AD (GB)**
  • **REIS, Nuno Miguel Fernandes
    Bath BA2 7AY (GB)**

(74) Representative: **Mewburn Ellis LLP
City Tower
40 Basinghall Street
London EC2V 5DE (GB)**

(56) References cited:
      **WO-A1-2012/123750     WO-A2-2011/143075
      GB-A- 2 408 961**

• **MASTICHIADIS C ET AL: "Capillary-based
immunoassays, immunosensors and DNA
sensors - steps towards integration and
multi-analysis", TRAC, TRENDS IN ANALYTICAL
CHEMISTRY, ELSEVIER, AMSTERDAM, NL, vol.
27, no. 9, 1 October 2008 (2008-10-01), pages
771-784, XP025559772, ISSN: 0165-9936, DOI:
10.1016/J.TRAC.2008.08.003 [retrieved on
2008-09-17]**
• **HENARES T G ET AL: "Single-step ELISA
capillary sensor based on surface-bonded
glucose oxidase, antibody, and
physically-adsorbed PEG membrane containing
peroxidase-labeled antibody", SENSORS AND
ACTUATORS B: CHEMICAL: INTERNATIONAL
JOURNAL DEVOTED TO RESEARCH AND
DEVELOPMENT OF PHYSICAL AND CHEMICAL
TRANSDUCERS, ELSEVIER S.A, CH, vol. 149, no.
1, 6 August 2010 (2010-08-06), pages 319-324,
XP027174100, ISSN: 0925-4005 [retrieved on
2010-06-25]**
• **LING YU ET AL: "Poly(vinyl alcohol)
Functionalized Poly(dimethylsiloxane) Solid
Surface for Immunoassay", BIOCONJUGATE
CHEMISTRY, vol. 18, no. 2, 1 March 2007
(2007-03-01), pages 281-284, XP055213329, ISSN:
1043-1802, DOI: 10.1021/bc060108p**

**EP 3 172 565 B1**

**Description**

**BACKGROUND TO THE INVENTION**

**Field of the invention**

The present invention relates to assays, methods for carrying out assays, assay kits and methods for manufacturing assay kits. The invention has relevance to capillary (especially microcapillary) assay technology.

**Related art**

A number of microfluidic assay platforms have been developed in last few decades including, microwell plates, microbeads, microcapillary/ microchannel and lateral flow (test strip) devices.

Microcapillaries and microchannels offer high surface area-to-volume ratios, short diffusion distances and the possibility of operating continuous flow assays. Multiple bands can be coated along the length of a microcapillary/microchannel by, for example, photolithographic or micro-syringe methods offering the possibility of simultaneous detection of a panel of analytes from a single sample feed (Mastichiadis et *al.,* 2002). More sophisticated chip devices can also be designed having a number of parallel microcapillaries or microchannels (Yacoub-George et *al.,* 2007) running from a single injection port. Samples can be loaded into the microcapillaries/ microchannels using syringes (Chin et *al.,* 2011), electrokinetics (Kawabata *et al.,* 2008) or centrifugal forces (Lee *et al.,* 2009). In the case of lateral flow devices, such as test strips (made from fleece) and paper-based lateral flow devices, samples can also be taken up into the device using capillary action (Gervais and Delamarche, 2009). Sin *et al.*, (2011) presents a detailed review of different types of microfluidic assay devices currently in development.

A wide range of assays can be performed in microcapillary and microchannel devices, including direct assays and agglutination assays which can be used, for example, for blood typing, the detection of pathogens or biomarkers, as well as immunoassays such as sandwich assays. Microcapillary and microchannel devices are mainly produced from glass and plastic materials.

Immunoassay devices which facilitate interrogation of the capillaries by matching the refractive index of the material from which the device is formed to the refractive index of the sample fluid, and thereby reducing adverse optical effects, are described in WO2011/117579 and Edwards et al. (2011). In these immunoassay devices, a population of first members of a respective specific binding pair is immobilised at least at a portion of the surface of the capillary bore. Consequently, these immunoassay devices are not suitable for conducting assays which require release of assay reagents, such as antibodies, from the capillary bore surface.

In addition to immobilization, assay reagents can also be preloaded inside microcapillaries and microchannels, using e.g. liquid reagents within blisters, dried reagent pouches, and so on (Madou *et al.,* 2001; van Oordt *et al.,* 2013).

Trapping of immunophenotyping reagents in a dried gelatine layer sandwiched between two glass slides has also been described (Beck et al., 2001; Beck et al., 2012 - WO2011/143075A2). However, this has not been applied to microcapillaries or microchannels. Furthermore, the volume of the gelatine layer available after surface coating of microcapillaries or microchannels would be extremely small, making it unlikely that a sufficient quantity of assay reagents could be trapped in such a gelatine layer to perform the assay. WO2011/143075A2 further makes it clear that the gelatine layer dissolves when a sample fluid is added. A device comprising such a gelatine coating therefore can only be used once. Embedding of assay reagents in a hydrogel is mentioned in Fujii *et al.* (2012), while Uchiyama *et al.* (2012) describes trapping of fluorescent substrates within a soluble PEG-based coating inside a microchannel. A hydrogel network inside microcapillaries which holds a covalently immobilised fluorescent substrate, and a second soluble coating containing an enzyme-labelled antibody is described in Wakayama *et al.* (2012). Gervais and Delamarche (2009) describe coating microchannels of a microfluidic chip for performing fluorescence immunoassays using Pluronic ® F108 to facilitate retention of antibodies and sample loading.

Flow through chips made of porous material such as silicon or glass functionalized with active chemical groups to allow antibodies to be immobilized on the channel walls have also been described (US2008/020453), as have devices ("platens") made of glass with an array of through-holes, wherein the interior walls of the through-holes may be coated with antibodies, target antigen, or with a hydrophilic coating (US2002/094533). WO03/042697 further describes a device ("assay-carrier") with through holes which may be hydrophilic and coated with antibodies.

Coating of non-porous fluorocarbon beads and stabilisation of the coating by cross-linking with glutaraldehyde is described in McCreath et al. (1997). Surface coating of plastic surfaces has also been described (Carneiro-da-Cunha et al., 2010; Huang et al., 2011; and Lin et al., 2005). The surface modification of flat hydrophobic substrates, including FEP, with high molecular weight polyvinylalcohol (PVA) has been described in Kozlov et al. (2003) and US 7,179,506 B2. However, flat substrates, like those described in Kozlov et al. (2003) and US 7,179,506 B2, are not suitable for use as assay devices, both because of their flat geometry and because there is no indication how assay reagents could be loaded onto such a surface. The coating techniques for flat substrates described in Kozlov et al. (2003) and US 7,179,506 B2, such as immersion in a PVA solution, are also not suitable for coating capillaries.

## SUMMARY OF THE INVENTION

The present inventors note that there is a lack of microcapillary assay devices which allow simple fluid handling in a similar way to membrane-based lateral flow technologies, which are able to take up fluids by capillary action. Simplifying fluid handling would not only simplify the operation of the microcapillary assay devices, as normally fluid handling equipment such as aspirators or syringes are needed for this purpose, but also reduce the cost of such devices.

Accordingly, the present inventors have devised the present invention in order to address this disadvantage.

As discussed above, microcapillary assay devices normally require syringes or aspirators for fluid handling, such as fluid uptake into the microcapillary.

Microcapillary assay devices are ideally made from thermoplastic plastics. However, optically clear thermoplastic materials, such as fluoropolymers, which allow detection of reactions through the microcapillary wall can be highly hydrophobic. As a result, microcapillaries or microchannels made from these materials require the use of high pressure differences to introduce aqueous sample liquids into the microcapillary or microchannel.

The present inventors have realised that very long plastic microcapillaries can be coated with a hydrophilic layer on the internal surface of the microcapillary without affecting the overall optical properties of the microcapillary wall (as example way of including a hydrophilic layer on the internal surface of a microcapillary bore). In particular, the present inventors realised that such a hydrophilic layer is effective in decreasing the contact angle of a sample fluid with the microcapillary wall and air, making the internal wall of the capillary or channel sufficiently hydrophilic for a sample fluid to be taken up into the microcapillary by capillary action. The possibility of coating the internal surface of microcapillaries, in particular microcapillaries made by melt-extrusion, such as fluorinated ethylene propylene (FEP) microcapillaries, is surprising as the surface of the capillary bores is very rough, which means surface charge and properties of an FEP microcapillary are not the same as in the case of a flat FEP sheet. Furthermore, coating of fluoropolymer microcapillaries, such as FEP microcapillaries, with a hydrophilic layer requires solutions to be pumped through the capillaries - the capillaries cannot be coated by simply dipping capillaries into a solution because they are very hydrophobic therefore repel aqueous fluids.

In addition, the present inventors observed that assay reagents, such as antibodies, enzymes, enzyme substrates, and dyes, can be reversibly retained in or on the same hydrophilic layer, so that when one end of the microcapillary is brought into contact with a sample fluid, the sample quickly rises in the capillary due to capillary forces and the assay reagent retained in the hydrophilic layer diffuses into the sample fluid from the hydrophilic layer. This is particularly valuable for assays in which mixing of the sample with the assay reagent is required, including blood typing. The present inventors also noted that when the optical properties and geometry of the microcapillary were favourable to direct optical interrogation, qualitative and quantitative assays could be performed in the microcapillaries in a reduced amount of time, with the result being detectable either by naked eye or using simple optical detection devices such as charge-coupled devices (CCD) or complementary metal-oxide-semiconductor (CMOS) digital image sensors.

The inventors also observed that the velocity of liquid rising in the microcapillary can be manipulated by controlling the shape and internal diameter of the microcapillary and/or modifying the wetting properties of hydrophilic layer. The wetting properties can be modified by choosing the type of polymer to be used in the coating process, and has a direct impact in the contact angle of the fluid with the capillary wall. In situations where there are two or more microcapillaries, the liquid sample rises simultaneously through all the capillaries allowing duplicate assays and/or different assays to be performed in the microcapillaries through the selection of appropriate assay reagents retained in the hydrophilic layer of the capillaries. This is relevant, for example, in blood typing, where the agglutination of red blood cells needs to be tested for a panel of antibodies against different blood group antigens. As used herein, the term retention and retaining includes, but is not limited to, adsorption and/or deposition of assay reagents on the hydrophilic layer.

Accordingly, in a first preferred aspect of the invention, there is provided an assay device having:

a unitary body with an exterior surface, the unitary body being substantially transparent to visible light and formed from a material having a refractive index in the range 1.26 to 1.40, the refractive index being measured at 20 °C with light of wavelength 589 nm, and
at least two capillary bores extending internally along the unitary body, wherein at least a portion of the surface of each capillary bore includes a hydrophilic layer for retaining an assay reagent, and
wherein the hydrophilic layer is also substantially transparent to visible light to allow optical interrogation of the capillary bores through the capillary wall.

Preferably, at least a portion of the surface of each capillary bore is coated with a hydrophilic layer for retaining an assay reagent. Preferably, an assay reagent is retained at least at a portion of the hydrophilic layer or hydrophilic layer-coated surface of one or more capillary bores of the assay device.

As mentioned above, the present inventors realized that a hydrophilic layer is also advantageous for retaining assay reagents. The retention is preferably reversible in that an assay reagent retained at the hydrophilic layer is released when the hydrophilic layer is brought into contact with a sample fluid. This is allows the assay reagent to diffuse into the sample fluid thereby allowing the detection of a substance of interest present in the sample fluid. For example, where the sample fluid is whole blood, an antibody retained at the hydrophilic layer can diffuse out from the hydrophilic layer and into the blood sample and cause agglutination of red blood cells in the blood sample where the red blood cells have the cognate antigen of the antibody on their surface.

It is noted here that the retention of an assay reagent by the hydrophilic layer may stand as an independent aspect of the present invention. Accordingly, in one aspect, the present invention provides an assay device having:

a unitary body with an exterior surface, the unitary body being substantially transparent to visible light and formed from a material having a refractive index in the range 1.26 to 1.40, the refractive index being measured at 20 °C with light of wavelength 589 nm, and
at least one capillary bore extending internally along the
unitary body,
wherein at least a portion of the surface of the capillary bore includes a hydrophilic layer and,
wherein an assay reagent is retained at least at a portion of the hydrophilic layer of the capillary bore, and
wherein the hydrophilic layer is also substantially transparent to visible light to allow optical interrogation of the capillary bores through the capillary wall.

Preferably, at least a portion of the surface of each capillary bore is coated with a hydrophilic layer for retaining an assay reagent. In addition, or alternatively, to one or more assay reagents being reversibly retained at the hydrophilic layer, one or more assay reagents may be irreversibly retained at the hydrophilic layer. For example, the irreversibly retained reagent(s) may be covalently bound to the hydrophilic layer. This allows, for example, sandwich immunoassays to be performed using the assay device by covalently attaching a capture antibody to the hydrophilic layer and optionally reversibly retaining a detection antibody at the hydrophilic layer. For example, an assay reagent, such as an antibody, may be irreversibly retained at the hydrophilic layer, such as using cross-linking, e.g. using glutaraldehyde or by covalent immobilisation chemistry well known to those skilled in the art.

It can often be difficult to reliably interrogate capillary bores known for use in assay techniques. This is due primarily to adverse optical effects. For example, when viewing a capillary bore from one direction, the light from close to the lateral sides of the bore tends to be subject to a greater degree of refraction than the light from the centre of the bore.

The optical signal to be optically interrogated is generated during the assay, and this almost always takes place in an aqueous solution/suspension. Forming a microcapillary assay device from a material having a refractive index close to that of water, namely a refractive index in the range 1.26 to 1.40, the refractive index being measured at 20 °C with light of wavelength 589 nm as set out above, allows these adverse optical effects to be avoided to the extent that allows a significant improvement in optical interrogation of the capillary bore. Preferably, the refractive index of the material is within plus or minus 0.07, more preferably 0.06, most preferably 0.05, of the refractive index of the sample fluid when measured at 20 °C with light of wavelength 589 nm. For example, in the case where the sample fluid is aqueous, a preferred lower limit for the refractive index of the material of the body of the device is 1.28. A preferred upper limit for the refractive index of the material of the body of the device is 1.38. The refractive index is measured at 20 °C with light of wavelength 589 nm. Most preferably, the refractive index of the material of the body of the device is substantially identical to the refractive index of the sample fluid. Such an assay device is described in WO2011/117579 and Edwards et al. (2011).

The refractive index of water is 1.33 (when measured at 20°C with light of wavelength 589 nm, corresponding to the yellow doublet sodium D line). For comparison, the refractive index of some other materials commonly used in the manufacture of microcapillary assay devices under the same conditions are: fused silica 1.46; poly (ether urethane) 1.49; poly (methyl methacrylate) 1.49; poly (vinyl alcohol) 1.50; polyethylene 1.51; low density polyethylene 1.51; polyethylene terephthalate 1.57-1.58; polystyrene 1.59; poly (vinyl chloride) 1.54.

The present inventors have also found that the hydrophilic layer can be chosen so that it does not substantially change the overall optical properties of the capillary wall (e.g. hydrophilic layer-coated capillary wall) compared with, for example, a non-coated capillary wall.

In another aspect, the present invention provides a method of performing an assay for determining the presence of a substance of interest in a sample fluid using a device of the present invention, the method including the steps:

Providing a sample fluid in the capillary bore(s) of the device, thereby bringing the sample fluid into contact with assay reagent(s) retained at the hydrophilic layer or hydrophilic layer-coated surface of the capillary bore(s); and optically interrogating the capillary bores through the capillary wall to determine the presence of the substance in the sample fluid. Providing a sample fluid in the capillary bore(s) of the device preferably comprises bringing a sample fluid into contact with the capillary bores of the device, and allowing the sample fluid to rise in the capillary bores by capillary action. Alternatively, providing a sample fluid in the capillary bore(s) of the device preferably comprises bringing a sample fluid into contact with the capillary bores of the device and allowing the sample fluid to fill the capillary bores by gravity. Preferably, bringing the assay reagent into contact with a substance of interest results in a measurable change, and the presence of the substance in the sample fluid is determined from the measured change. When the sample fluid is allowed to fill the capillary bores by gravity, it is preferred that the length of the capillary bore is sufficient to maintain most of the capillary bore filled with sample fluid. This may be achieved using known balancing of liquid pressure head with capillary forces.

The method may be a method of detecting the presence of a microorganism of interest in a sample fluid. There are multiple ways in which the presence of a microorganism in a sample fluid may be detected. For example, the assay reagent may be a substrate for an enzyme produced by the microorganism of interest. In this case, reaction of the enzyme with the substrate may result in a measureable change, and the presence of microorganism in the sample fluid may be determined from the measured change. Alternatively, the assay reagent may be a substrate metabolised by the microorganism of interest, whereby metabolism of the substrate by the microorganism may result in a measureable change, and the presence of microorganism in the sample fluid may be determined from the measured change. In an alternative example, the assay reagent may be a substance which inhibits or promotes the growth of the microorganism of interest. In this case, increased or decreased growth, or inhibition of growth, of the microorganism of interest may result in a measurable change, and the presence of microorganism in the sample fluid may be determined from the measured change. As a further alternative, binding of the assay reagent to the microorganism of interest may result in a measurable change, and the presence of the microorganism in the sample fluid may be determined from the measured change. The microorganism may, for example, be a bacterium or a fungus, such as yeast. Preferably, the microorganism is a bacterium.

Alternatively, the method may be a method of performing a blood typing assay using a device of the present invention, the method including the steps:

providing a blood sample in the capillary bore(s) of the device; and
optically interrogating the capillary bores to determine if agglutination of red blood cells has taken place in the capillary bores;
wherein the presence of agglutination in a capillary bore indicates that the red blood cells have the antigen, with which the antibodies are capable of specifically binding, on their surface. Providing a blood sample in the capillary bore(s) preferably comprises bringing a blood sample in contact with the capillary bores of the device, and allowing the blood to rise in the capillary bores by capillary action.

Reliable interpretation of results is of utmost importance when determining a patient's blood type because of e.g. the dangers involved in transfusing incompatible blood into a patient. In addition, blood-typing tests should be fast and easy to use to allow identification of a patient's blood type "at the bedside", which can of vital importance in emergency situations. Cost efficiency is also an important consideration. The assay devices of the invention fulfil all of these requirements.

Preferably, a sample fluid (e.g. an aqueous solution or aqueous suspension, such as whole blood) rising in the capillary bores by capillary action results in release of the assay reagent retained at the hydrophilic layer. The released assay reagent preferably diffuses into the sample fluid. The hydrophilic layer is preferably resistant to dissolution in the presence of a sample fluid, in other words a sample fluid rising in the capillary bores by capillary action preferably does not result in dissolution of the hydrophilic layer.

The present inventors have also realised that low cost manufacture of disposable, capillary-based assay devices, such as blood typing devices, can be difficult to achieve. Accordingly, the inventors have devised a manufacturing method that allows ease of production of capillary assay devices pre-coated with a hydrophilic polymer layer and optionally also pre-loaded with an assay reagent. The inventors have realised that it is possible to coat, as well as load the first members of a specific binding pair into a long length of a capillary body (e.g. 20 cm or longer), the capillary body subsequently being cut to the desired length for an assay device. The unitary body of the device may be an extruded body, preferably a melt-extruded body. The device may be a disposable device.

Accordingly, in another aspect, the present invention provides a method for manufacturing an assay device of the present invention, the method including:

providing an extruded body having at least two capillary bores extending internally along the body, the body being substantially transparent to visible light and formed from a material having a refractive index in the range 1.26 to 1.40, the refractive index being measured at 20 °C with light of wavelength 589 nm; inserting a coating fluid into the capillary bores to coat at least a portion of the surface of each capillary bore with a hydrophilic layer for retaining assay reagents, wherein the hydrophilic layer is also substantially transparent to visible light; and forming a coated extruded body.

Preferably, the method further comprises the step of

inserting a respective loading fluid into one or more capillary bores of the coated extruded body, each loading fluid comprising an assay reagent, to retain the assay reagent(s) at least at a portion of the hydrophilic layer-coated surface of the capillary bore(s), and
forming a coated and loaded extruded body.

The present inventors have realised that use of a loading fluid with high viscosity increases the retention of assay reagent at the hydrophilic layer by forming a thin film layer at the wall. The loading fluid may have a viscosity of at least 1.3 mPa ·s, at least 1.5 mPa ·s, at least 1.7 mPa ·s, at least 2 mPa ·s, at least 4 mPa ·s, at least 6 mPa ·s, at least 8 mPa ·s, at least 10 mPa ·s, at least 20 mPa ·s, at least 30 mPa ·s, at least 40 mPa ·s, at least 50 mPa ·s, or at least 60 mPa ·s when measured at 20°C. Preferably, the loading fluid has a viscosity of at least 6 mPa ·s when measured at 20°C. Most preferably, the loading fluid has a viscosity of at least 60 mPa ·s when measured at 20°C. Methods for measuring viscosity are well known in the art and available to the skilled person. For example, the loading fluid may comprise at least 10%v/v, at least 20%v/v, at least 30%v/v, at least 40%v/v, at least 50%v/v, at least 60%v/v, at least 70%v/v, or at least 80%v/v glycerol. Preferably, the loading solution comprises at least 50%v/v glycerol. Most preferably, the loading solution comprises at least 80%v/v glycerol. The viscosity of a 50%v/v glycerol solution is 6.00 mPa ·s when measured at 20°C, while the viscosity of a 80%v/v glycerol solution is 60.1 mPa ·s when measured at 20°C.

The step of inserting the loading fluid may be achieved by injection of the loading fluid into the capillary or suction of the gas from the capillary in order to draw the loading fluid into the capillary. In one embodiment, the capillary is substantially filled along its length with loading fluid before excess loading fluid is removed from the capillary bore. In these embodiments, the loading fluid may form a loading fluid slug filling 50%, 60%, 70%, 80%, 90% or more, or substantially all of the volume of the capillary bore. In an alternative embodiment, only part of the capillary bore is filled with loading fluid before excess loading fluid is removed from the capillary bore. In these embodiments, a loading fluid slug can be drawn along the capillary bore drawn along the capillary bore loading the assay reagent onto the device. In these embodiments, the loading fluid may form a loading fluid slug filling less than 50%, 40% or less, 30% or less, 20% or less, 10% or less, or 5% or less of the volume of the capillary bore. Both methods can efficiently deposit assay reagents on the assay devices of the present invention. The method preferably further includes the step of removing excess loading fluid. The step of removing excess loading fluid may be achieved by injection of the gas into the capillary or aspiration of the loading fluid from the capillary. Inert gas may be injected into the capillary bore after excess loading fluid is removed (or as part of the removal of the excess loading fluid). Such injection of gas can dry the interior of the capillary bore having assay reagent deposited thereupon.

Preferably, the method further includes the step of cutting the coated, or coated and loaded, extruded body to form the device for an assay of a required length, wherein the coated, or coated and loaded, extruded body, before cutting, optionally has a length of at least 20 cm.

Alternatively, the method may be a method for manufacturing a set of n devices, the method further including cutting the coated, or coated and loaded, extruded body to form the set of n devices, each device having a length of at least X, wherein the loaded extruded body, before cutting, has a length of at least nX, or a length of at least 20 cm.

In another aspect, the present invention provides an assay system for carrying out assays, the system having at least one assay device according to the present invention, and a holder for holding the plurality of assay device. The holder preferably provides observation means to allow at least a part of the assay device to be observed.

In a still further aspect, the present invention provides an assay kit including an extruded unitary body having at least two capillary bores extending internally along the body, the unitary body being substantially transparent to visible light and formed from a material having a refractive index in the range 1.26 to 1.40, the refractive index being measured at 20 °C with light of wavelength 589 nm, and the unitary body further having a length of at least 20 cm, wherein at least a portion of the surface of each capillary bore is coated with a hydrophilic layer for retaining an assay reagent, the extruded unitary body being capable of being cut to a required length for an assay. Preferably, an assay reagent is retained at least at a portion of the hydrophilic layer or hydrophilic layer-coated surface of each capillary bore.

The kit may be provided in the form of a reel of the coated, or coated and loaded, extruded unitary body.

Further preferred (or at least optional) features are set out below. Unless the context demands otherwise, these can be combined either singly or in any combination with any aspect of the invention. Similarly, any aspect of the invention can be combined with any other aspect of the invention.

Preferably, in use the contact angle ($\theta$) of the sample fluid present in the capillary bores with the hydrophilic layer and air is less than 90 degrees, more preferably less than or equal to 85 degrees, less or equal to 80 degrees, less or equal to 75 degrees, less than equal to 70 degrees, or less or equal to 65 degrees. Most preferably the contact angle ($\theta$) of the sample fluid with the hydrophilic layer and air is less than or equal to 65 degrees. The sample fluid may be an aqueous solution or aqueous suspension, such as whole blood.

Where the capillary bores are circular, the contact angle (θ) is preferably measured by bringing one end of a capillary bore in contact with a sample fluid (e.g. an aqueous solution or an aqueous suspension, such as whole blood) and measuring the maximum height (h) the sample fluid reaches in the capillary bore and calculating the contact angle (θ) using the (Young-Laplace) equation

$$h = 4\gamma cos(\theta)/\rho g d,$$

wherein γ is the surface tension of the fluid, ρ is the density of the fluid in g/cm$^3$, g is the gravitational acceleration and d is the mean internal diameter of the capillary. The density of the fluid is preferably measured at 20 °C.

Where the capillary bores are non-circular (e.g. elliptical or oval), the contact angle (θ) is measured by bringing one end of a capillary bore in contact with a sample fluid (e.g. an aqueous solution or an aqueous suspension, such as whole blood) and measuring the maximum height (h) the sample fluid reaches in the capillary bore and calculating the contact angle (θ) using the equation

$$h = (2\gamma cos(\theta)/\rho g) * (1/a+1/b)$$

wherein γ is the surface tension of the fluid, ρ is the density of the fluid in g/cm$^3$, g is the gravitational acceleration, a is the minor axis (conjugate diameter) of the capillary and b is the major axis (transverse diameter) of the capillary. The density of the fluid is preferably measured at 20 °C.

Alternatively, the contact angle (θ) can be measured by pumping the sample fluid (e.g. whole blood) through a capillary bore at a constant mean superficial flow velocity and taking a snapshot of the liquid-air front with a microscope or other imaging equipment, and the wetting angle, $\theta_1$ is determined from the tangent of the meniscus at the contact point of the liquid with the capillary wall. As a further alternative, the whole meniscus can be fitted with a semi-circle and in this case $\theta_1$ can be determined using trigonometry rules. The procedure is then repeated for recessing flow, and $\theta_2$ determined. The "dynamic" wetting angle in capillaries is prone to hysteresis; so the contact angle θ is taken as the mean value of $\theta_1$ and $\theta_2$.

Preferably, the thickness of the hydrophilic layer is such that it does not substantially alter the overall optical properties, including the refractive index, of the capillary wall. The hydrophilic layer may be less than or equal to 20 μm thick. Preferably, the thickness of the hydrophilic layer is less than or equal to 15 μm, less than or equal to 10 μm, less than or equal to 5 μm, less than or equal to 4 μm, less than or equal to 3 μm, less than or equal to 2 μm, less than or equal to 1 μm, less than or equal to 900nm, less than or equal to 800 nm, less than or equal to 700nm, less than or equal to 600nm, less than or equal to 500 nm, less than or equal to 400nm, less than or equal to 300nm, less than or equal to 200 nm, or less than or equal to 100nm. More preferably, the thickness of the hydrophilic layer is less than or equal to 500 nm, or less than or equal to 300 nm, most preferably less than or equal to 300nm. In addition, or alternatively, the thickness of the hydrophilic layer is preferably at least 10nm, at least 20nm, at least 30nm, at least 40nm, at least 50nm, at least 60nm, at least 70nm, at least 80nm, at least 90nm, or at least 100nm. Thickness may refer to the mean, or median, thickness of the hydrophilic layer.

The hydrophilic layer may be porous, preferably micro-porous.

Preferably, the hydrophilic layer comprises, or consists of, a polymer such as a water-soluble polymer. The hydrophilic layer may comprise, or consist of, polyvinylalcohol (PVA), preferably cross-linked polyvinylalcohol (PVA); a cellulose derivative, such as hydroxypropylmethylcellulose (HPMC) or carboxymethyl cellulose; collagen; poly-lysine (poly-D-lysine, poly-L-lysine, or a combination thereof); gelatine; PVA and dextrin; PVA and dextran; PVA and gelatine; cross-linked chitosan; or alginate and calcium, and mixtures thereof. Preferably, the hydrophilic layer comprises, or consists of, cross-linked PVA, gelatine, HPMC, PVA and dextrin or PVA and dextran. More preferably, the hydrophilic layer comprises or consists of cross-linked PVA. Most preferably, the hydrophilic layer comprises or consists of cross-linked low molecular weight PVA. Hydrophilic layers, such as hydrophilic layers comprising or consisting of PVA or chitosan, can be cross-linked using glutaraldehyde, for example.

The PVA may be a low molecular weight PVA. For example, the PVA may have a molecular weight of at least 10,000 g/mol, at least 11,000 g/mol, at least 12,000 g/mol, or at least 13,000 g/mol. Preferably, PVA has a molecular weight of at least 13,000 g/mol. In addition, or alternatively, PVA may have a molecular weight of up to 25,000 g/mol, up to 24,000 g/mol, or up to 23,000 g/mol. Preferably, PVA has a molecular weight of up to 23,000 g/mol. The molecular weight of PVA in this context refers to the molecular weight of PVA prior to any cross-linking.

Alternatively, the PVA may be a high molecular weight PVA. The PVA may have a molecular weight of at least 100,000 g/mol, at least 110,000 g/mol, at least 120,000 g/mol, at least 130,000 g/mol, or at least 140,000 g/mol. Preferably, PVA has a molecular weight of at least 140,000 g/mol. In addition, or alternatively, PVA may have a molecular weight of up to 210,000 g/mol, up to 200,000 g/mol, or up to 190,000 g/mol. Preferably, PVA has a molecular weight of up to 190,000 g/mol. Again, the molecular weight of PVA in this context refers to the molecular weight of PVA prior to any cross-linking.

Preferably, the surface of the capillary bore coated with the hydrophilic layer is substantially evenly, or substantially uniformly, coated with the hydrophilic layer. In other words, the hydrophilic layer is preferably substantially evenly, or substantially uniformly, distributed on the portion of the capillary bore coated with the hydrophilic layer. Without wishing to be bound by theory, a substantially even or uniform coating is thought to improve uptake of sample fluids by the capillary by capillary action. A substantially even, or substantially uniform, coating may refer to a continuous or a discontinuous hydrophilic layer. Preferably, the hydrophilic layer is continuous.

Alternatively, the hydrophilic layer comprises, or consists of a surfactant. Examples of surfactants include, but are not limited to, polyethylene glycol ethers, oleic acid, alkyl sulfonates, alkylsuccinamates, and polyethylene oxide.

The hydrophilic layer is suitable for retaining an assay reagent. "Retaining" or "retention", as referred to herein, is preferably reversible. Preferably, retention of an assay reagent by the hydrophilic layer is reversed when the hydrophilic layer is brought into contact with a sample fluid, e.g. an aqueous solution, or an aqueous suspension, such as whole blood. In use, an assay reagent retained by the hydrophilic layer is made available to a sample fluid present in the capillary bore. Thus, where an assay reagent is retained at least at a portion of the hydrophilic layer or hydrophilic layer-coated surface of each capillary bore, said assay reagent is made available to a sample fluid present in the capillary bore. Where the hydrophilic layer comprises a polymer, an assay reagent may, for example, be retained by the hydrophilic layer through entrapment within the polymer chains and released by diffusion when brought into contact with a sample fluid.

As mentioned above, the hydrophilic layer may be cross-linked. Cross-linking may increase the hydrophilicity of the hydrophilic layer, thereby increasing the speed by which a sample brought into contact with one end of the capillary bore is taken up into the capillary bore by capillary action. The layer may, for example, be cross-linked by means of a chemical cross-linking agent, heat, or ultraviolet radiation. Exemplary chemical cross-linking agents include glutaraldehyde and paraformaldehyde, and bi-functional reactive cross-linkers such as bis-epoxy polyethylene glycol. Non-covalent cross-linking methods may also be used, such as using hydrogen bonding between polymer chains, electrostatic interactions between polymer chains (e.g. positively charged polymer cross-linked with negatively-charged polymer), cross-linking using a divalent ion, particularly a divalent cation (e.g. calcium cross-linking of alginate polymer).

Preferably, in use a sample fluid brought into contact with one end of the capillary bores rises in the capillary bores by capillary action.

In addition to being substantially transparent to visible light, the material of the body of the device may also be substantially transparent to electromagnetic radiation in the invisible spectrum, e.g. ultraviolet (UV) light.

Preferably the unitary body is formed from a hydrophobic material. The hydrophobic material is preferably a polymer, most preferably a fluoropolymer. The fluoropolymer may be fluorinated ethylene propylene (FEP), tetrafluoroethylene hexafluoropropylene vinylidene fluoride (THV), perfluoroalkoxy (PFA), polytetrafluoroethylene (PTFE), ethylene tetrafluoroethylene (ETFE), or poly(chlorotrifluoroethylene) (PCTFE). Most preferably, the unitary body is formed from FEP.

The capillary bores may have an inner diameter of at least 10 $\mu$m. Preferably, the inner diameter is at least 50 $\mu$m. The inner diameter may be up to 1 mm. More preferably, the inner diameter is 100 to 500 $\mu$m, most preferably 100-200 $\mu$m. For example, the inner diameter of the capillary bores may be 100 $\mu$m, 200 $\mu$m, 300 $\mu$m, 400 $\mu$m, or 500 $\mu$m. Where the capillary bores are elliptical or oval, the capillary bores may have a minor axis (conjugate diameter) of at least 10 $\mu$m. Preferably, the minor axis is at least 50 $\mu$m. The minor axis may be up to 1 mm. More preferably, the minor axis is 100 to 2500 $\mu$m, most preferably 100-200 $\mu$m. For example, the minor axis of the capillary bores may be 100 $\mu$m, 200 $\mu$m, 300 $\mu$m, 400 $\mu$m, or 500 $\mu$m. The smaller the diameter or minor axis of the capillary bores, the higher a sample fluid will rise in the capillary bores by capillary action. This can be calculated according to the Young-Laplace equation presented above. Where the minor axis and the major axis differ substantially (i.e. a<<b), the minor axis controls the liquid height in the capillary.

The maximum fluid height in the capillary bore and the velocity of sample fluid rising in capillary bore can be controlled by the diameter and shape of the capillary and by the hydrophilic layer, as both of these factors influence the contact angle of the sample fluid with the hydrophilic layer or hydrophilic layer-coated capillary bore surface and air.

A small inner diameter or minor axis for the capillary bores is also advantageous as assay reagents, such as antibodies, retained in or at the hydrophilic layer require time to diffuse and react with the sample fluid rising in a capillary bores by capillary action. For example, the time for diffusion of an assay reagent, such as an antibody (assuming D = $1\times10^{-10}$ m$^2$ s$^{-1}$) in a capillary bore with a diameter or minor axis of 200 $\mu$m is less than 100 s, where D is the diffusivity of the molecule through water. This allows, for example, an agglutination reaction to take place in the capillary bore in the same time scales that it takes blood to rise in the capillary bore by capillary action (typically 30 to 120 s). The fast release of assay reagents from the hydrophilic layer in combination with the very short diffusion times thereby allow reactions to take place between the assay reagent and a substance of interest present in a sample fluid as the sample fluid rises in the capillary bore by capillary action.

The cross sectional shape of the capillary bores may be circular. However, more preferably it is oval or elliptical, in view of the preferred manufacturing technique for the device. In that case, the "inner diameter" is to be taken as the minor axis of the capillary bore in cross section.

The device may have more than two capillary bores formed in the unitary body. For example, the device may have 3, 4, 5, 6, 7, 8, 9, 10 or more capillary bores. It is possible to manufacture a suitable device with 20 capillary bores, or more.

Preferably, the capillary bores are formed substantially parallel to each other.

Preferable, an assay reagent is retained at least at a portion of the hydrophilic layer or hydrophilic layer-coated surface of one or more capillary bores.

One capillary bore in the device may have a differently-treated surface from at least one other capillary bore in the device. This may provide a measurable difference in assay performance between the bores. For example, one bore may have a different assay reagent, or a different concentration of assay reagent, retained at the hydrophilic layer or hydrophilic layer-coated surface of the capillary bore than another bore.

An assay reagent, as referred to herein, may be any substance suitable for detecting or determining the presence of a substance of interest in a sample. Preferably, bringing the assay reagent in contact with a substance of interest directly, or indirectly, results in a measurable change. The measurable change may, for example, be a change in colour, a change in fluorescence, or agglutination of the substance of interest.

For example, the assay reagent may be selected from the group consisting of: a population of first members of a respective binding pair, each first member being capable of specifically binding with a second member of the respective binding pair; an enzyme; an enzymatic substrate; a dye; a pH indicator (such as a pH indicator dye); a substance which inhibits or promotes growth of a microorganism (such as a bacterium); particle reagents (such as microparticles, nanoparticles and microbeads, including but not limited to latex particles, gold nanoparticles and fluorescence beads); and a metabolic substrate for a microorganism (such as a bacterium).

Where the assay reagent is a population of first members of a respective binding pair, one bore in the assay device may have first members of a different specific binding pair, or a different concentration of first members, retained at the hydrophilic layer or hydrophilic layer-coated surface of the capillary bore compared with another bore.

In some embodiments, at least one reference capillary bore may be provided without said assay reagent retained at the hydrophilic layer or hydrophilic layer-coated surface of the capillary bore. The reference bore may be treated with a reference protein, such as BSA. Furthermore, it is possible for two or more capillary bores to have the same assay reagent retained at the hydrophilic layer or hydrophilic layer-coated surface of the capillary bore, in order to provide measurement redundancy in the device. In this case the capillary bores are said to have received identical treatment.

Still further, it is possible for two or more capillary bores to receive identical treatment and for one or more other bores in the same device to receive different treatment, to provide combinations of these advantages.

Preferably, an assay device as referred to herein is a portable assay device, e.g. a hand-held assay device.

An assay device, as referred to herein, may be an assay device for carrying out a blood typing assay. The population of first members may a population of antibodies, and said antibodies may be capable of specifically binding with an antigen present on the surface of some types of red blood cells thereby causing agglutination of said red blood cells. The red blood cells are preferably human red blood cells. Similarly, blood or whole blood, as referred to herein, is preferably human blood or human whole blood. A preferred assay device is for determining an individual's ABO blood type and comprises at least two capillary bores, wherein one bore has anti-A antibodies retained at the hydrophilic layer or hydrophilic layer-coated surface of the capillary bore and at least one other bore has anti-B antibodies retained at the hydrophilic layer or hydrophilic layer-coated surface of the capillary bore. The assay device may have a further capillary bore, wherein said capillary bore has anti-D antibodies retained at the hydrophilic layer or hydrophilic layer-coated surface of the capillary bore for determining whether an individual is Rhesus positive or Rhesus negative.

Alternative or additional antibodies which can be retained in the hydrophilic layer are antibodies specific for Rh, Kell, Kidd, Duffy, MNS, P, Lewis, or Lutheran blood system antigens. Antibodies for blood typing are commercially available, for example from Alba Bioscience. Preferably, antibodies as referred to herein are monoclonal antibodies.

"Agglutination" refers to the clumping together of particles, e.g. red blood cells, suspended in a liquid in the presence of an antibody or other first binding member of a specific binding pair capable of binding more than one second binding member of the specific binding pair present on the particles simultaneously. By binding multiple particles, the antibody or other first binding member joins them, creating a larger complex.

Preferably, the exterior surface of the body includes a measurement first surface and a measurement second surface. In use, it is intended that light will be transmitted through the device from the measurement first surface to the measurement second surface. These surfaces may, for example, be upper and lower major surfaces of the body. Preferably one or both of the measurement first surface and the measurement second surface extend substantially parallel with the principal axes of the capillary bores. One or both of the measurement first surface and the measurement second surface may extend substantially parallel with the arrangement direction of the capillaries.

One or both of the measurement first surface and the measurement second surface may be substantially planar.

The advantage of this is that optical distortions due to refraction at the measurement surfaces can be reduced or avoided. In turn, this can improve the signal-to-noise ratio of a measurement taken by optical interrogation of the capillary bores. Note that typically the body also includes side surfaces. The shape of the side surfaces is not considered to be critical, since preferably optical interrogation of the capillary bores does not take into account light from or close to the side surfaces.

Preferably, the length of the coated, or coated and loaded, extruded body, before cutting, is at least 50 cm. The length may be greater, e.g. at least 1 m, preferably at least 2 m, 3 m, 4 m, or 5 m. Most preferably the length of the extruded body, before cutting, is at least 5 m.

The assay may be performed including an optical interrogation step. Preferably, this is performed to provide a pixellated image of one or more, or all, of the capillaries. For example, a digital camera, such as a charge-coupled device (CCD) camera, digital microscope, or flatbed scanner may be used. Generally, charge-coupled imaging devices or devices comprising metal-oxide-semiconductor (CMOS) digital image sensors are preferred.

In the case of a blood-typing assay, when blood is brought into contact with an assay device of the present invention, the blood rises in the capillary bores by capillary action until it reaches the maximum height possible under the particular conditions in the absence of agglutination. This is referred to herein as $h_{max}$. If the antibodies present in the hydrophilic coating of a capillary bore cause agglutination of the red blood cells, the flow of the blood in the capillary bore is retarded, with result that the blood does not reach $h_{max}$. Thus, agglutination can be detected by measuring the grey-scale intensity along a short section of the (or each) capillary near $h_{max}$. For the capillaries where the blood did not agglutinate (and therefore was capable of reaching $h_{max}$) the plot will reveal a strong signal corresponding to strong light absorption, whereas for the capillaries where the red blood cells have agglutinated the plot will show no signal compared to background because of the absence of red blood cells in the area scanned. Alternatively, agglutination can be detected by plotting the greyscale along a short section, preferably 5 mm or less in length, of the (or each) capillary at a reference distance, e.g. $h_{max}/2$. In this case, agglutination of red blood cells results in high signal variability, whereas non-agglutinated blood presents as a very homogenous colour intensity plot along the imaging section.

Alternatively optical interrogation may be by direct optical observation, i.e. using the naked eye. In this case, a short section of the capillary, preferably 5 mm or less in length, may be viewed across the microcapillary array at a reference distance, e.g. $h_{max}/2$, and the result of assay optically detected. For example, in the case of a blood-typing assay, agglutinated blood would present as patches of red blood cells. In the case of such direct optical observation, a magnifying device may be used, such a plastic magnifier or magnifying glass.

Matching of the refractive index of body of the device to that of the sample fluid is advantageous both for digital (e.g. using a digital camera or flatbed scanner) and naked-eye detection of assay reactions.

An assay system may have a plurality of assay devices of the invention, and the holder may be for holding the plurality of assay devices. Preferably, in such an assay system, the holder holds the assay devices in a substantially planar array. Furthermore, preferably the holder provides observation means (such as an observation window) to allow at least a part of the (or each) assay device to be observed. For example, the observation means may allow the (or each) assay device to be observed at $h_{max}$ and/or $h_{max}/2$. The observation means may also allow each assay device to be optically interrogated for measurement.

The holder preferably also allows the assay to proceed whilst the assay device(s) are held in the holder.

The system may further include a tray having an arrangement of wells adapted to receive reagents, sample fluids or other liquids required for the assay. The tray is preferably further adapted to receive at least an end of the (or each) assay device when the assay device(s) are held in the holder. This allows one end of each capillary bore to be in fluid communication with a liquid held in the respective well.

The term binding pair, as used herein, refers to a first member and a second member which are capable of specifically binding with one another. Examples of binding pairs include antigen-antibody. A first member of a specific binding pair may, for example, be an antibody. A second member of a specific binding pair may be an antigen, e.g. an antigen present on the surface of some red blood cells which is indicative of a particular blood type.

The term "antibody" describes an immunoglobulin whether natural or partly or wholly synthetically produced. The term also covers any polypeptide or protein comprising an antibody antigen-binding site. Thus, this term covers antibody fragments, derivatives, and chimeric molecules comprising an antibody antigen-binding site, or equivalent, fused to another polypeptide (e.g. derived from another species or belonging to another antibody class or subclass).

Antibody fragments that comprise an antibody antigen-binding site include, but are not limited to, antibody molecules such as Fab, Fd, Fv, dAb, isolated CDR regions, F(ab')2, Fab', Fab'-SH, scFv, bispecific single chain Fv dimmers; and diabodies. Such antibody fragments are well known in the art.

An antigen-binding site as referred to herein is the part of a molecule that binds to and is complementary to all or part of the target antigen. In an antibody molecule it is referred to as the antibody antigen-binding site, and comprises the part of the antibody that binds to and is complementary to all or part of the target antigen. Where an antigen is large, an antibody may only bind to a particular part of the antigen, which part is referred to as an epitope. An antibody antigen-binding site may be provided by one or more antibody variable domains. An antibody antigen-binding site may comprise an antibody light chain variable region (VL) and an antibody heavy chain variable region (VH).

An antigen as referred to herein is any substance which can specifically bind with an antibody. Such substances include: proteins, peptides, polysaccharides, nucleic acids, and small molecules (e.g. haptens). Antigens may be proteins, carbohydrates, glycoproteins, and glycolipids, in particular, proteins, carbohydrates, glycoproteins, and glycolipids present on surface of some types of red blood cells. Detection of such antigens allows determination of an individual's blood type, or the measurement of antigen dissolved in a biological sample.

The terms "specific" and "specifically" as used herein may refer to the situation where one member of a specific binding pair will not show any significant binding to molecules other than its specific binding partner(s), e.g. the other member of the specific binding pair. These terms also apply where e.g. an antigen binding domain is specific for a particular epitope which is carried by a number of antigens, in which case the specific binding member carrying the antigen binding domain will be able to bind to the various antigens carrying the epitope.

The term "comprise" is generally used herein in the sense of include, i.e. permitting the presence of one or more additional features or components.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

Certain aspects and embodiments of the invention will now be illustrated by way of example and with reference to the figures described below.

## BRIEF DESCRIPTION OF THE DRAWINGS

Fig. 1 is a schematic diagram of an extrusion apparatus for use in manufacturing assay devices according to a preferred embodiment of the invention.

Fig. 2 is a schematic cross section through the die shown in Fig. 1.

Fig. 3 is a schematic view from below of the die shown in Fig. 1.

Figs. 4A-D illustrate the effect of refractive index of the body of the capillary assay device on optical distortions during optical interrogation.

Fig. 5A summarises the variation of the signal and noise with the refractive index for MCF-FEP (Fluorinated Ethylene Propylene microcapillary film) and MCF-EVA (ethylene vinyl acetate microcapillary film) .

Fig. 5B summarises the variation of signal-to-noise ratio in MCF-FEP and MCF-EVA.

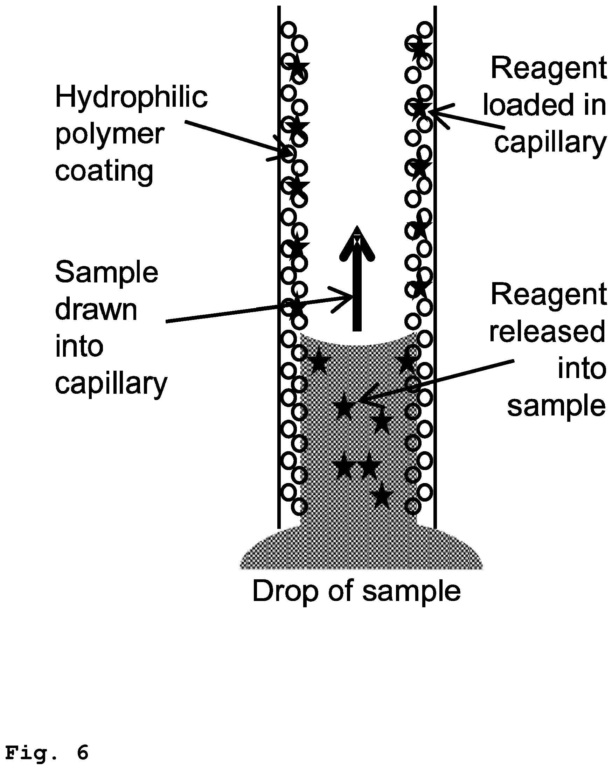

Fig. 6 shows a liquid sample being drawn by capillary action into an FEP microcapillary pre-coated with a hydrophilic polymer and pre-loaded with an assay reagent. Assay reagents are released by fluid convection and rapid molecular diffusion in the small diameter microcapillary.

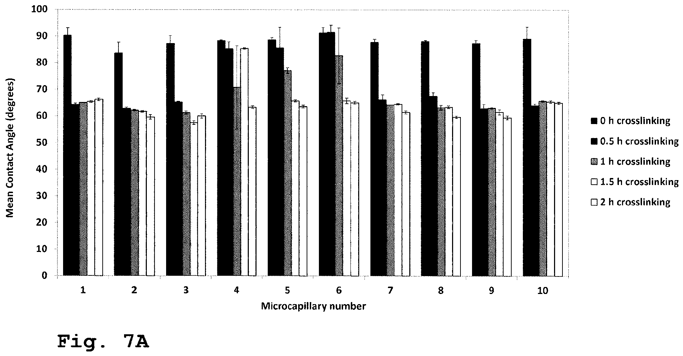

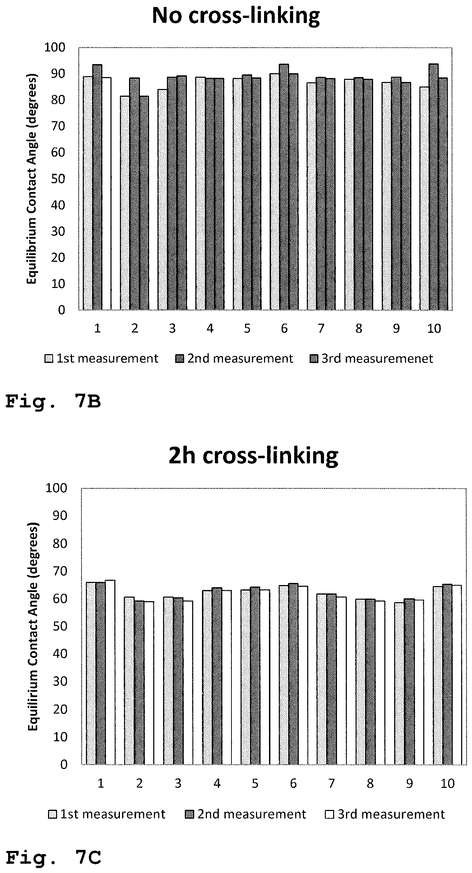

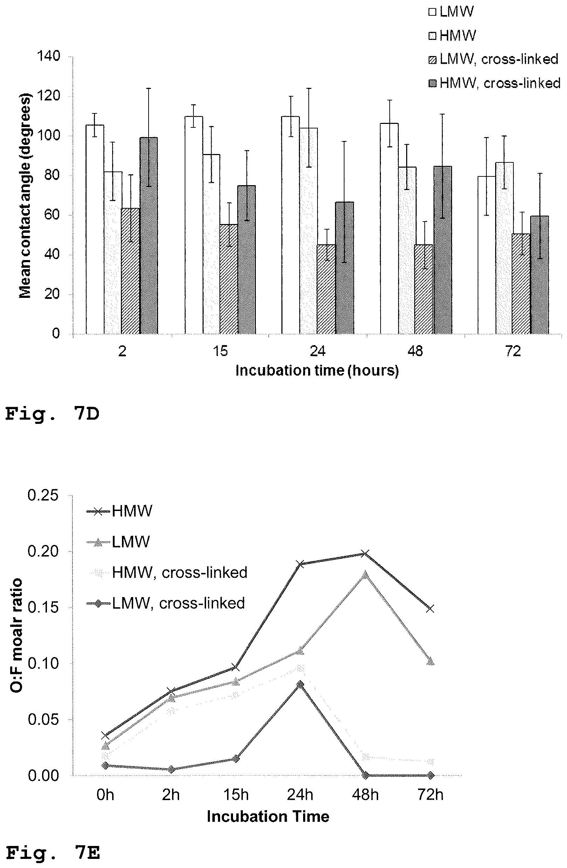

Fig. 7A shows that the mean contact angle in a 10 bore FEP microcapillary film can be reduced by coating the microcapillaries overnight with low molecular weight PVA, and cross-linking for 0.5 to 2 hours. Figs. 7B and 7C show that the equilibrium contact angle does not change when the microcapillary is used multiple times, demonstrating that the coating is stable both when cross-linked and when not cross-linked. Fig. 7D shows mean contact angle of capillaries with respect to the time the capillaries were incubated with the coating solution. Capillaries were coated with cross-linked or uncross-linked low molecular weight (LMW) or cross-linked or uncross-linked high molecular weight (HMW) PVA. Fig. 7E shows x-ray photoelectron spectroscopy data of capillaries coated with cross-linked or uncross-linked low molecular weight (LMW) or cross-linked or uncross-linked high molecular weight (HMW) PVA with respect to the time the capillaries were incubated with the coating solution.

Fig. 8 shows the capillary rise (in cm) in FEP microcapillaries coated with different hydrophilic polymer layers.

Fig. 9 shows tapping-mode 10*10 $\mu$m Atomic Force Microscopy images of the internal surface of an FEP microcapillary coated with gelatine (polymer B), HPMC (polymer C) and PVA (polymer A) cross-linked with glutaraldehyde. The reduction in contact angle is related to the surface coverage of the coating, with cross-linked PVA being the most effective at reducing the contact angle. An even coating is advantageous for achieving consistent capillary rise.

Fig. 10 shows FITC-labelled antibody (IgM) retained in the cross-linked PVA coating inside a dried microcapillary (left-hand panel). When the capillary is partly filled, a high concentration of antibody is transported through the capillary by the liquid at the liquid-air interface (central panel). In the filled capillary, the antibody diffuses out of the cross-linked PVA coating into the capillary volume (right-hand panel).

Fig. 11 shows that IgG and IgM molecules (which have different molecular weights) are released at different rates into the capillary volume from a cross-linked PVA coating.

Figs.12-14 illustrate a method of manufacturing an assay device according to an embodiment of the present invention. Fig. 12 shows bulk immobilisation of blood typing reagents in a long microcapillary array.

Fig. 13 shows cutting of long pre-coated capillary array with antibody solution immobilised on the inner wall of the capillaries to produce individual test strips.

Fig. 14 shows fabrication of multiplex dip-stick for instantaneous blood typing from a drop of blood.

Fig. 15 shows release of antibody solution from the hydrophilic layer retained on the microcapillary wall.

Fig. 16A shows results of anti-A, anti-B and anti-D (Rhesus) tests from an A+ blood type in pre-coated a hydrophilic microcapillary film produced from FEP.

Fig. 16B shows the greyscale profile plot showing high greyscale variability and a decrease in red blood cells concentration along the film, representing a positive agglutination test with anti-A.

Fig. 16C shows the greyscale profile plot showing reduced greyscale variability and constant red blood cells concentration along the film, representing a negative agglutination test with anti-B.

Fig. 16D shows the greyscale profile plot showing high greyscale variability and a decrease in red blood cells concentration along the film, representing a positive agglutination test with anti-A.

Fig. 17A shows the result of an anti-A test for an A+ blood type in a dip-stick format.

Fig. 17B shows the separation of red blood cells from blood serum upon positive blood agglutination (illustrated with anti-A test to an A+ blood type).

Fig. 17C shows the greyscale profile plot showing the separation of the plasma/buffer from the red blood cells along a capillary.

Fig. 18A shows multiplex blood grouping tests in pre-coated MCF-FEPs for different blood types (A+, B+ and O+). The capillaries were coated according to the coating pattern shown in Fig. 12.

Fig. 18B shows direct detection (i.e. naked-eye detection) of agglutination in the capillaries coated according to the coating pattern shown in Fig. 12.

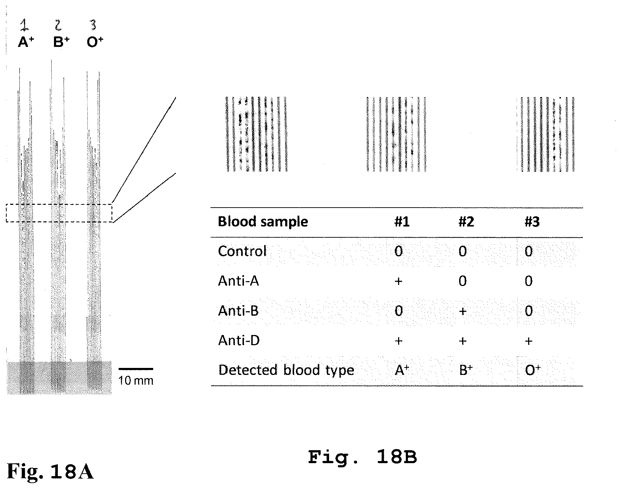

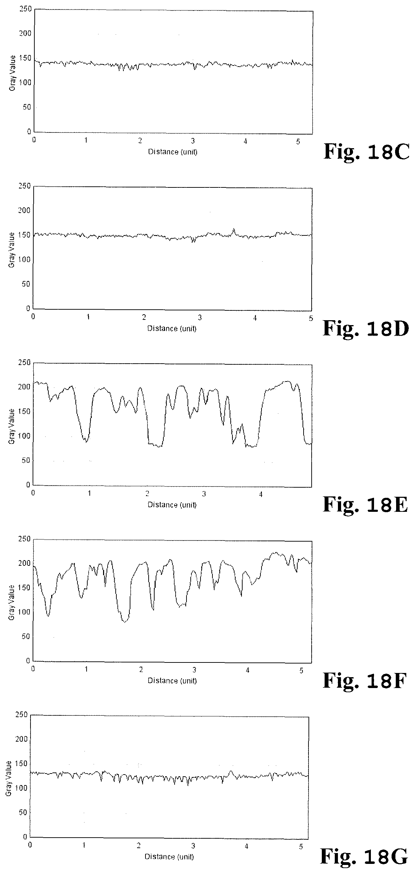

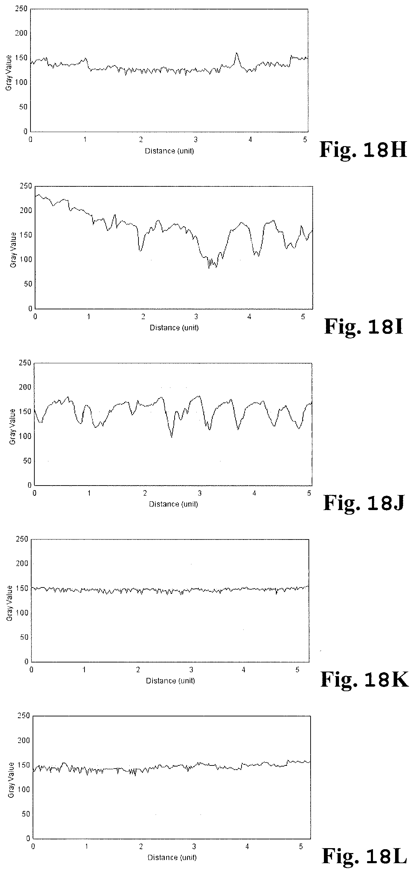

Figs. 18C-L show greyscale profile plots along the capillaries in the imaging section of blood sample 1 in Fig. 18B. Specifically, Fig. 18C shows the greyscale profile plot along capillary 1 in imaging section of blood sample 1 in Fig. 18B. Fig. 18D shows the greyscale profile plot along capillary 2 in imaging section of blood sample 1 in Fig. 18B. Fig. 18E shows the greyscale profile plot along capillary 3 in imaging section of blood sample 1 in Fig. 18B. Fig. 18F shows the greyscale profile plot along capillary 4 in imaging section of blood sample 1 in Fig. 18B. Fig. 18G shows the greyscale profile plot along capillary 5 in imaging section of blood sample 1 in Fig. 18B. Fig. 18H shows the greyscale profile plot along capillary 6 in imaging section of blood sample 1 in Fig. 18B. Fig. 18I shows the greyscale profile plot along capillary 7 in imaging section of blood sample 1 in Fig. 18B. Fig. 18J shows the greyscale profile plot along capillary 8 in imaging section of blood sample 1 in Fig. 18B. Fig. 18K Greyscale profile plot along capillary 9 in imaging section of blood sample 1 in Fig. 18B. Fig. 18L shows the greyscale profile plot along capillary 10 in imaging section of blood sample 1 in Fig. 18B.

Fig. 19 shows blood rising in capillary coated with PVA by capillary action. Either BSA (control), anti-A, anti-B, or anti-D by capillary action is retained in the PVA coating, as indicated. This figure demonstrates the very fast kinetics of an assay performed in an assay device of the present invention.

Fig. 20 shows the results of a sandwich ELISA performed in FEP microcapillaries coated with cross-linked low molecular weight PVA.

Fig. 21 shows a schematic of a method for efficient deposition of assay reagents in the inner surface of the coated capillaries.

Fig. 22A shows liquid deposition in the wall of a 200 micron internal diameter microcapillary coated with cross-linked HMW PVA. Fig. 22B shows percentage reduction in slug length with capillary number for a 10mm long water slug.

Fig. 23 shows a schematic of an alternative method for efficient deposition of assay reagents in the inner surface of the coated capillaries, which consists of loading the entire length of the capillary with the assay reagent dissolved in a fluid, and then removing excess assay reagent.

Fig. 24 shows imaged strips of enzymatic and fermentation assays, allowing colorimetric identification of different bacteria strains.

Fig. 25 shows a schematic of an assay device for immunoassays consisting of immobilising a set of capture antibodies or antigens on the hydrophilic coating, and then interfacing a strip with a small microwell in which sample and other reagents are deposited, and assay fluidics run by capillary action.

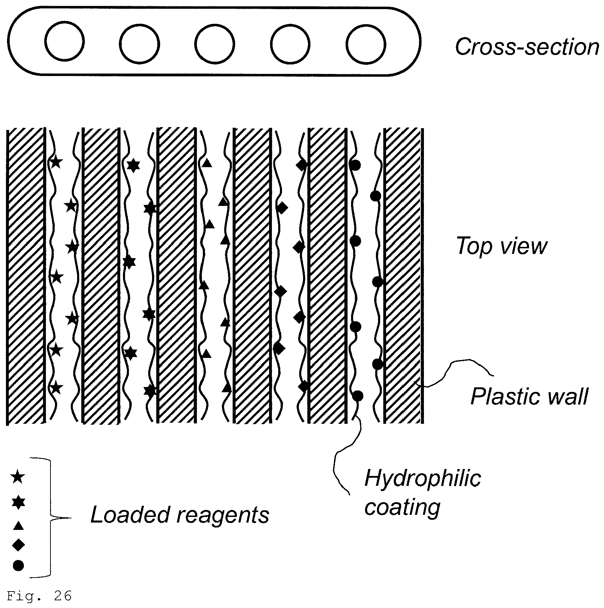

Fig. 26 shows a schematic showing the concept of loading different assay reagents in an array of capillaries.

## DETAILED DESCRIPTION OF THE PREFERRED EMBODIMENTS, AND FURTHER PREFERRED FEATURES OF THE INVENTION

In a preferred embodiment of the present invention, there is provided a platform for assays. The present inventors have shown the internal surface of capillaries can be coated with a hydrophilic layer to facilitate retention of assay reagents, and to increase the rate at which fluid samples are taken up by the capillaries through capillary action.

The array is preferably an array of 10 microcapillaries, each capillary having an elliptical cross section and a minor axis or conjugate diameter of 100-200 $\mu$m, embedded in a single substantially flat plastic film extruded from a fluoropolymer material.

The flat geometry and excellent optical properties of the plastic film allows direct cross-interrogation of the capillaries for signal detection using either the naked eye or conventional optical systems, such as a CCD camera or a flatbed scanner. The small internal volume and diameter of the capillaries shortens the time required for assay reagents retained in the hydrophilic layer to diffuse and react with any substances of interest present in the capillary.

This new assay platform finds major application in a number of different assays, including, for example, as a clinical tool for the determination of a patient's blood type, and in the detection of bacteria in a sample fluid to perform serological tests or immunoassays, e.g. for quantification of biomarkers.

In a preferred embodiment, the present invention utilises a capillary body manufactured in accordance with the disclosure of WO 2005/056272, the content of which is hereby incorporated by reference in its entirety.

WO 2005/056272 discloses apparatus for producing an extrudate product, the extrudate product including a plurality of capillary channels therethrough, the apparatus comprising an extruder having an inlet, a die including an orifice having a predetermined outer shape, a plurality of needles each having a body including an internal conduit for fluid flow, each needle further comprising an outlet from the internal conduit at an outlet end, the outlet end of each needle being arranged in a predetermined pattern substantially within the orifice of the die, the conduit of each needle being fluidly connected to a fluid source, wherein, in use:

a) extrudable material is fed into the extruder through the inlet;

b) the extruder melts and forces the extrudable material around the bodies of the needles towards the die and through the orifice in the die to produce an extrudate product having substantially the predetermined outer shape;

c) the needles allow fluid to be drawn from the fluid source through the conduit to be entrained in the extrudate product to form capillaries such that the extrudate product includes capillaries therealong in the predetermined pattern.

It has been found that the problem of die swell within the capillary is substantially reduced or negated when fluid is allowed to enter the capillary. This allows the bores of the capillaries to be more accurately controlled so small bore capillaries can be reliably produced. It is envisaged that capillaries having a bore of between about 2 mm to 10 microns may be produced in a single stage of melt processing. However, it is envisaged that a further processing stage could produce capillaries having a bore of below 1 micron. It should be understood that the capillary bores are also referred to as microcapillaries.

It is preferred that the needle outlets are substantially regularly distributed in the die orifice as this helps to prevent maldistribution of the extrudate. It is preferred that each needle outlet is a substantially equal distance from other outlets and from the orifice of the die. For example, if the die orifice is substantially rectangular and the predetermined pattern of needle outlets is a simple line of outlets within the orifice it is preferred that the line is arranged substantially centrally in the short side of the rectangle and that the distances between the needle outlets are substantially identical to the distance between the outer needle outlets and the short edges of the orifice, and the line of outlets and the long edges of the orifice. The needle outlet may be any suitable size, but it is preferably between 2 mm and 0.1 mm and most preferably between 0.6 mm and 0.2 mm. For instance, with a needle outlet size of 0.3 mm capillary bores of between 200 microns and 20 microns can be readily produced depending on the processing conditions.

It is preferred that the pressure of the fluid entering the capillaries through the needles is substantially equal to the pressure of the environment into which the extrudate product is being extruded as it has been found that this produces a more stable extrudate product. It is preferred that the flow of extrudable material entrains the fluid in the capillary, but it should be understood that the fluid may enter the capillaries at above or below the pressure of the environment into which the extrudate product is being extruded, but that greater control may be needed. The fluid allowed to enter the capillaries will typically be air at room temperature and pressure, but the extrusion may be in a liquid bath or other non-typical environment. The fluid source may be air at room temperature and pressure if the extrudate product is being extruded into such an environment and can be drawn straight from the local atmosphere. However, it should be understood that the fluid source may be an inert gas or liquid, or a sample gas or liquid that is to be trapped within the capillaries in the extrudate product.

It is preferred that a gear pump is used to steady the flow of extrudable material between the extruder and the die. This helps to reduce any flow abnormalities that may result from variations in the operation of the extruder.

The die is used to take the feed of material from the extruder and change the shape of the material flow until it has the desired outer shape and can exit though the die orifice which has substantially the predetermined outer shape. It should be understood that, due to die swell, the outer shape of the extrudate may not correspond exactly with the predetermined shape of the orifice. It is preferred that the die is a converging die. The die is preferably shaped to ensure that the flow over the needles is substantially even as this helps to create a well formed, regular extrudate.

It is preferred that the die orifice is substantially rectangular so the resulting outer shape of the extrudate product is substantially rectangular. The dimensions of the rectangular orifice are preferably such that the extrudate product is a sheet or film. Preferably the rectangular orifice has a long side having a length that is at least times longer than the short side. Preferably the ratio is greater than 10 as this may allow the film to flex more readily. It should be understood that the orifice could take any other suitable shape, including an annulus, square or circle. It has been noted that with a non-circular die, for instance a rectangular die there may be edge effects that alter the shape of the capillaries at or near an edge of the film. Such edge effect may be negated through the use of an annular die which is, in effect, a continuous film having no edges. An annular die may allow the production of an extrudate product have greater consistency in the size and shape of the capillaries.

For simplicity the apparatus will now be described with reference to a preferred embodiment in which the die has a substantially rectangular orifice in which an array of needle outlets are arranged in a line substantially parallel with the long side of the rectangle and substantially in the centre of the short sides of the orifice. This produces an extruded film having a plurality of capillaries therealong.

It should be understood that different arrays and orifice shapes could be employed.

It is preferred that the needle outlets are substantially circular in shape. This shape of outlet is easy to form, but other shapes could be used if desired. It is also preferred that the body of each needle is substantially cylindrical and is elongate along a first axis. The bodies are preferably arranged such that the first axis of the cylindrical body is substantially parallel with the flow of material as this provides a low resistance to the material flow and is simple to manufacture.

It should be understood that the plurality of needles may be formed individually, integrally, or in groups of two or more needles. For example a solid monolith of metal could be used to form a plurality of needles. The monolith may include holes therethrough to form the needles required by the invention. The needles may include a common inlet which then divides into a plurality of conduits leading to a plurality of outlets. The outlets of the needles from the monolith may protrude from the monolith allowing the extrudate to flow around the protrusion before gas is drawn from the outlet, or there may be no protrusion. The extrudate will flow around the monolith and draw gas through the outlets as described above.

Although it has been mentioned above that die swell within the capillaries is substantially reduced or negated, die swell still occurs at the die exit. The outer shape of the extrudate product will swell as it exits the orifice. In the case of the film, it has been found that the swell is greater along the short axis of the rectangular orifice than along the long axis. The result is that the substantially circular capillaries within the extrudate prior to swelling are distorted into an elliptical shape with the long axis substantially parallel to the short axis of the rectangular cross section of the film.

It should be understood that with variations in outlet shape and processing, the capillaries cross section can be varied.

The extrudate product is preferably drawn away from the orifice at a rate greater than the rate at which the product is produced. The draw ratio is the ratio of the rate of production of extrudate to the rate at which the extruded product is drawn off. At some draw ratios (between 16 and 20) it appears that the die swell effect dominates and the capillaries are substantially elliptical.

At higher draw ratios (above 30) the change in geometry due to the extrudate drawing dominates. As has been shown in the literature, during drawing of an extrudate having a rectangular cross section, the length of the short axis decreases at a faster rate than the length of the long axis of the extrudate and so the capillaries are distorted to form substantially elliptical capillaries that have their long axis substantially parallel to the long axis of the rectangular cross section. The drawing process typically reduces the overall cross sectional dimensions of the extrudate product and therefore reduces the dimensions of the capillaries within the product.

It has also been found that it may be possible to further process the extrudate product after drawing. This further processing can be either cold drawing or warm drawing at an elevated temperature. It has been found that cold drawing can reduce the product dimensions by between two and three times and a greater reduction is to be expected when warm drawing is used.

The apparatus and a process using the apparatus is capable of producing rectangular section extrudate product with multiple capillaries running along the length of the product.

WO 2005/056272 discloses the production of extrudates with elliptical multiple capillaries of major axis length roughly 65 $\mu$m and minor axis length of about 35 $\mu$m. It should be noted that the aspect ratio and the mean diameter of the capillary can be varied through changes in the process conditions. The extruded products typically take the form of films. Each film typically has a length and a substantially rectangular cross section perpendicular to said length, said cross section including two long sides and two short sides, the film including a plurality of capillary bores substantially parallel to the length of the film.

WO 2005/056272 further discloses that the production of a length of extrudate of about 20 m long allowed an investigation of the dimensions of the capillaries at five sections along the extrudate via scanning electron microscopy. This revealed that the variation in the dimensions of the capillaries was no greater than about 10% along the length of the product.

WO 2005/056272 still further discloses the formation of extruded products using, for example LLDPE. Such polymers are found to have good optical transparency, despite any crystalline content present within the polymer. WO 2005/056272 suggests that total, or at least a significantly increased level of, optical transparency could be achieved by using an amorphous polymer such as polystyrene. However, it is to be noted that these materials are not necessarily the preferred materials for use with the embodiments of the present invention.

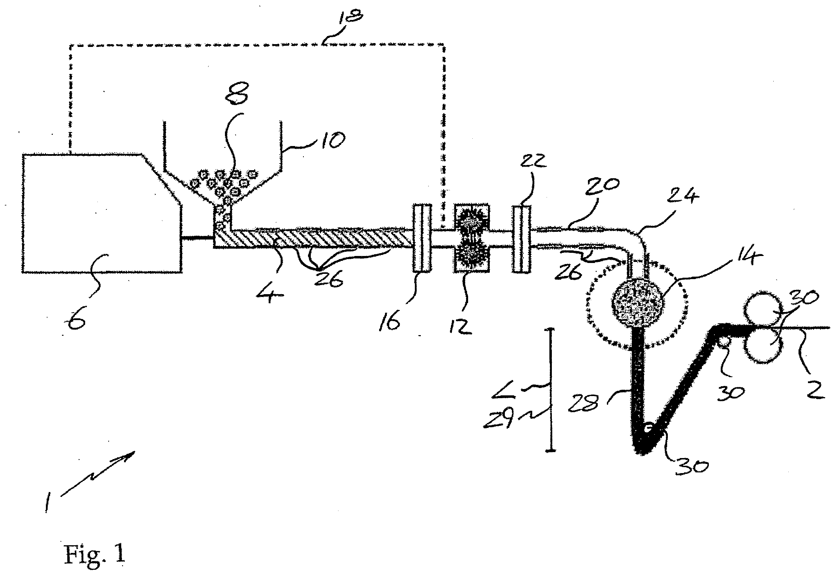

Fig. 1 shows extrusion apparatus 1 for creating an extrudate product 2 having capillary bores therealong. The extrusion apparatus comprises screw extruder 4 driven by a motor 6. Extrudable material 8 is fed to the extruder screw 4 through a hopper 10. As the extrudable material passes through the extruder screw 4 the material is melted to form a melt (not shown). The extruder screw 4 feeds the melt to a gear pump 12 which maintains a substantially constant flow of melt towards a die 14. The gear pump 12 is connected to the extruder screw 4 by a flange 16 which includes a screen filter to remove impurities from the melt flow. The motor 6 is controlled using a pressure feedback link 18 between the inlet of the gear pump and the motor 6.

The melt passes to the die 14 through an extruder barrel 20 which is connected to the gear pump by a flange 22. In this embodiment the extruder barrel includes a 90° bend 24. Band heaters 26 are used to control the temperature at different stages in the extrusion apparatus 1. Band heaters 26 may be located within the extruder, on the flanges 16,22, on the gear pump 12, on the extruder barrel 5 20 and also on the die 14.

The detail of the arrangement of the die 14 will be shown in greater detail in subsequent figures.

The melt passes through the die 14 and is formed into the desired shape and cross section. As the melt passes out of the die it becomes an extrudate 28. The extrudate 28 is drawn down over and between rollers 30. The drawing down process, as described above, alters the cross section of the extrudate 28 to form the extrudate product 2. A draw length (L) 29 is defined between the orifice and the first roller 30. It has been found that L has a great effect on the extrudate product 2 formed by this apparatus.

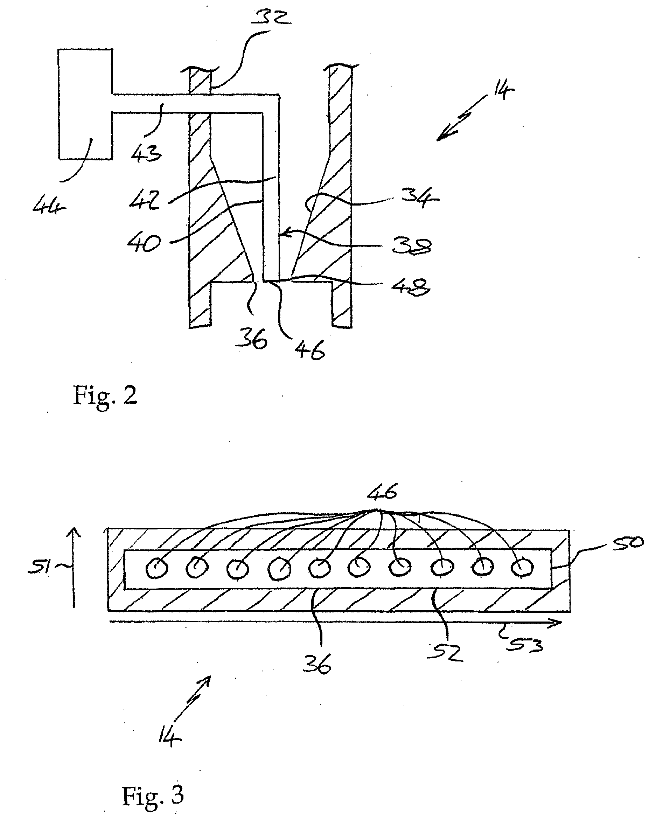

Fig. 2 shows a schematic cross section through the die 14 of Fig. 1. The die includes an entry portion 32, a convergent portion 34 and an orifice 36 which has a predetermined outer shape. The melt enters the entry portion 32 of the die 14, is gradually shaped by the convergent portion 34 until the melt exits the orifice 36.

The die 14 further includes needles 38 (only one of which is shown in this figure) positioned therein. The needle 38 a body portion 40 having a conduit 42 therein which is fluidly connected to a fluid source 44 by means of a second conduit 43 passing through a wall of the die 14 around which the melt must flow to pass to the orifice 36. The needle 38 further includes an outlet 46 at an end 48 of the needle 38. The needle 38 is arranged such that the outlet 46 is located within the orifice 36.

Fig. 3 shows a schematic view of the die 14 from below. This drawing shows that the orifice 36 has a rectangular outer shape. The orifice has a short side 50 substantially parallel with a short axis 51 and a long side 52 substantially parallel with a long axis 53.

In this example, the die includes ten needles 38 with the outlets 46 distributed substantially evenly along the long axis 53 within the orifice and substantially centrally in orifice along the short axis 51. In this example, the die orifice has a short side dimension of 1.5 mm, a long side dimension of 18mm and the needles have a 0.5 mm outer diameter and a 0.3 mm inner bore.

In an example process, a polymer melt is produced in a screw extruder 4 and its resultant flow rate stabilised by means of a gear pump 12. This melt is then fed into a die 14 in the orifice of which is arranged a plurality of outlets from needles 38 in a predetermined pattern. A conduit 42 through each needle 38 is fed from a horizontally orientated feed conduit 43, the entrance of which is open to atmosphere outside of the die which is the fluid source 44. The resulting extrudate is then passed over a series of rollers 30 into a haul-off device (not shown). The speed of the haul-off device can be altered so that extrudate products 2 with differing draw ratios can be produced.

The die 14 is designed such that the incoming flow from the extruder, which is contained in a circular pipe, is altered such that it may pass through the orifice 36 of the die 14. The die 14 must effect this geometry change, and this is currently achieved by using a convergent die 14.

The die 14 is also designed so that the flow over the array of needles 38 is substantially even. An even melt flow around the needles 38 facilitates creation of well formed extrudate 28. If, however, there is an uneven flow, the melt will preferentially channel along a path of least resistance. This results in a distorted extrudate 28.

In WO 2005/056272, the process is operated at about 165°C using linear low density polyethylene (LLDPE). The motor 6 is controlled using a pressure feedback loop that is set to 300 PSI and this, in turn, causes a pressure of around a few bar in the die 14. Air is entrained as a result of the polymer flow over the array of needles 38 and the feed to this needle 38 array is left open to the atmosphere. The velocity of the polymer melt at the die orifice 36 is of the order of one centimetre per second, the velocity of the haul off device can be set anywhere between zero and 9 metres per minute.

The parameter that was found to have substantial influence on the final product was the distance L 29, shown in Fig. 1 and defined to be the distance between the die exit and the first roller 30. In fact, in this case the first roller is a stationary polished stainless steel rod submerged in a water bath.

The effect of variation of L is explained in further detail in WO 2005/056272.

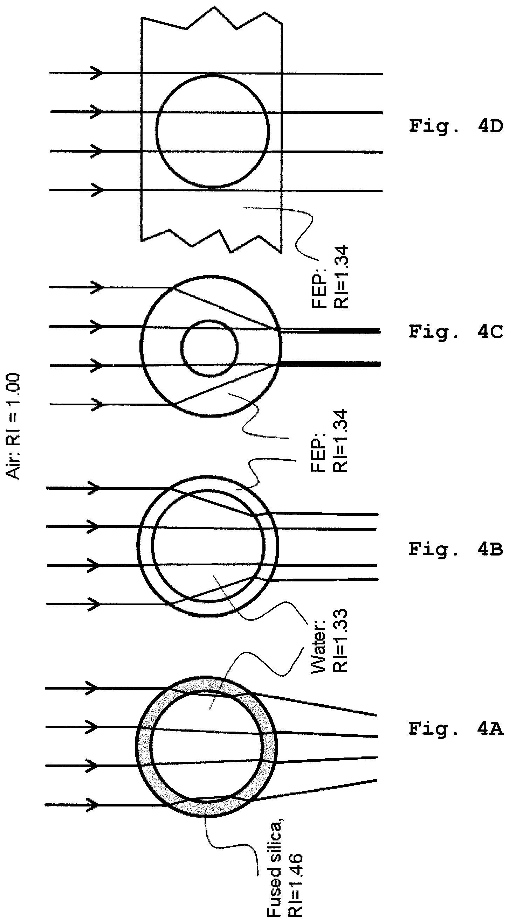

One drawback of many known capillary-based assays relates to optical interrogation of the capillary bore. This is illustrated in Fig. 4A-D.

Figs. 4A -shows a typical capillary device formed from fused silica. Fig. 4B shows a capillary produced from FEP material. An aqueous sample fluid (water in this case) is located in the capillary bore. As shown in Fig. 4A, refraction of light transiting the device occurs at the interface between the air and the body of the device and also at the interface between the body of the device and the sample fluid in the bore. This is due to changes in refractive index at those interfaces. The refractive index of air is 1.0, the refractive index of water is 1.33 and the refractive index of fused silica is 1.46. The refractive index of blood serum when measured at 20 °C with light of wavelength 589 nm is 1.33 to 1.36.

However, as shown in Figs. 4C and 4D, the effect of refraction at the interface between the body of the device and the bore can be reduced or even avoided by forming the body of the device using a material having a refractive index close to or the same as that of the sample fluid (water in this case). Results are expected to be similar where e.g. whole blood is used as the sample fluid, as the refractive index of blood serum is very similar to that of water. Suitable materials include Fluorinated Ethylene Propylene (FEP) with a refractive index of 1.34; Tetrafluoroethylene hexafluoropropylene vinylidene fluoride (THV) with a refractive index of 1.35; Perfluoroalkoxy (PFA) with a refractive index of 1.34; Polytetrafluoroethylene (PTFE) with a refractive index of 1.35 - 1.38; Ethylene Tetrafluoroethylene (ETFE) with a refractive index of 1.40; and Poly(chlorotrifluoroethylene) (PCTFE) with a refractive index of 1.39.

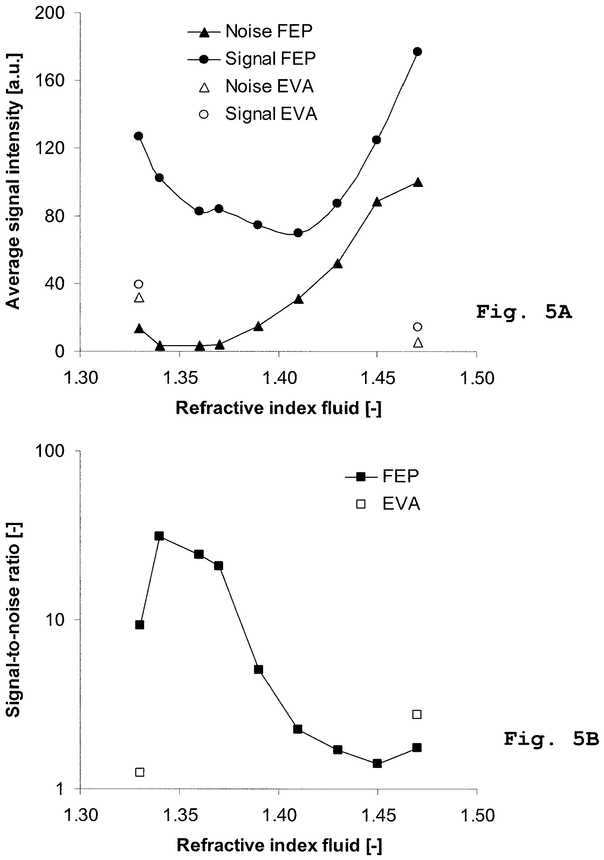

Fig. 5A summarises the variation of the mean peak height as determined from profile plots for colourless (noise) and blue dyed (signal) water-glycerol mixtures in MCF-FEP (fluorinated ethylene propylene, mean internal capillary diameter 206 $\mu$m, 10 capillaries, refractive index: 1.34) and MCF-EVA (ethylene vinyl acetate, mean internal capillary diameter 142 $\mu$m, 19 capillaries, refractive index: 1.48) at increasing refractive index of the fluid.

Fig. 5B summarises the variation of the mean signal-to-noise ratio in MCF-FEP and MCF-EVA at increasing refractive index of water-glycerol mixtures.

Fluorinated Ethylene Propylene (FEP) is considered to be particular suitable for assay applications because it has a refractive index very close to that of water. However, the use of fluoropolymers, such as FEP, has several drawbacks. Firstly, fluoropolymers, including FEP, are very hydrophobic, and therefore are not capable of taking up sample fluids by capillary action. Instead, sample fluids must be drawn into the capillary using e.g. aspirators or syringes, thereby complicating fluid handling and increasing the cost of such assay devices. In addition, assay devices made from fluoropolymers, such as FEP, cannot be used to carry out certain types of assays, such as blood-typing, as the hydrophobic nature of the material causes assay reagents, such as antibodies, to become immobilized on the capillary wall, thereby preventing them from diffusing out into a sample fluid present in the capillary. Diffusion of reagents is essential for many types of assays. For example, in blood-typing antibodies must be able to diffuse out of the hydrophilic layer in order to bind red blood cells and thereby cause their agglutination.

The present inventors have overcome these problems by coating the capillary wall internally with a hydrophilic layer.

Specifically, the inventors have shown that coating capillaries with a hydrophilic layer can decrease the contact angle of a sample fluid present in the capillary with the capillary wall, thereby facilitating uptake of samples into the capillary by capillary action.

In the experiments described herein, the equilibrium contact angle of the capillaries was determined by immersing dried microcapillaries in a transparent reservoir with deionised (DI) water, and recording the maximum liquid rise height, h, in the capillaries with respect to the free liquid surface in the reservoir. The capillaries were then dried again with gaseous nitrogen and the liquid height measured a second and a third time to assess stability of the hydrophilic coating.

The equilibrium contact angle, $\theta$ (in degrees) for circular capillaries with mean internal diameter, d was calculated based on Laplace-Young equation,

$$h = 4\gamma\cos(\theta) / \rho g d$$

assuming a surface tension, $\gamma$ for water-air of 72.8 mN/m and specific density, $p$ of water of 1,000 kg/m$^3$. g refers to the gravitational acceleration.

For elliptical capillaries, the minor axis of the capillary, a and major axis, b of the capillary were measured using image analysis of microphotographs of thin cross-sections of the microcapillaries, and the equilibrium contact angle, $\theta$ (in degrees) calculated based on a modified Laplace-Young equation:

$$h = (2\gamma \cos(\theta) / \rho g)*(1/a+1/b)$$

assuming a surface tension, $\gamma$ for water-air of 72.8 mN/m and specific density, $p$ of water of 1,000 kg/m$^3$. g refers to the gravitational acceleration.

First the contact angle of microcapillaries coated with PVA or cross-linked PVA was investigated. A 20 mg/mL solution of PVA with a low molecular weight (Mwt: 13,000-23,000) was pumped through a 10 bore microcapillary film fabricated from FEP having mean internal capillary diameter of 206 $\mu$m using a HPLC pump at a constant flow rate of 0.5mL/min. The PVA solution was allowed to recirculate overnight to ensure good adsorption of the polymer onto the microcapillary walls. In order to cross-link the PVA adsorbed onto the internal surface of the capillaries, a 0.5 mg/mL glutaraldehyde solution was recirculated for 30 min at 0.5 mL/min while heating the microcapillary film to 40°C. Cross-linking was then continued for up to further 2 hours following addition of 10 mL of a 5M HCl solution to the glutaraldehyde solution. The material was then washed with 500 ml of hot deionised water (60°C) and subsequently with 200 ml of ambient temperature deionised water. The microcapillary film was then dried using gaseous nitrogen.

The equilibrium contact angle after PVA coating was θ = 88±1.9 degrees. The equilibrium contact angle was found to drop rapidly with cross-linking time as shown in Figure 7A. After 2 hours of cross-linking, the contact angle was reduced to θ = 62±2.5 degrees (Fig. 7A). In contrast, the equilibrium contact angle for uncoated FEP microcapillaries was θ = 123±1.6 degrees. The contact angle values shown in Fig. 7 represent the mean of three contact angle measurements using the same capillaries, demonstrating that the PVA coating is stable and did not dissolve during the measurements.

The effect of incubation time, cross-linking and relative molecular weight of PVA on mean contact angle was then tested in a 10 bore FEP microcapillary film. Low molecular weight (LMW; 13,000-23,000 Da) and high molecular weight, (HMW; 146,000-186,000 Da) PVA were tested using PVA solutions with a concentration of 20 mg/ml. Incubation times were kept constant. Solutions were aspirated with the help of a syringe, and then strips incubated for the incubation time shown in the figures. Where relevant, polymer was cross-linked with glutaraldehyde using same protocol as described above. Error bars show 2*standard deviations between 3 consecutive liquid rise measurements across the 10 bore fluoropolymer strip. Fig. 7D shows that the LMW cross-linked PVA is most effective in reducing the contact angle, however it was possible to produce capillaries with hydrophilic coatings, i.e. capillaries with a mean contact angle lower than 90 degrees, with all LMW and HMW PVA coatings tested. Out of the coatings tested in this experiment, LMW PVA was the least effective at reducing the mean contact angle which is believed to be related to dissolution of the LMW PVA coating deposited on the capillary surface and possibly the fact that the coating is thinner (see also x-ray photoelectron spectroscopy [XPS] data in Fig. 7E). Dissolution of the coating results in an increase in the contact angle when consecutive measurements are taken and thus in large standard deviations for the contact angle measurements. Overall, all coatings tested were effective in producing hydrophilic FEP capillaries. The XPS data shown in Fig. 7E supports the deposition of the polymer on the surface of the FEP microcapillaries (which translates into an increase in the oxygen:fluorine [O:F] molar ratio), which varies with incubation time. The O:F molar ratio provides the molar ratio between the oxygen and fluorine groups (which includes CF2 and CF3) deposited on the fluoropolymer surface. The O:F molar ratio does not differentiate thickness from surface coverage, but is an estimate of the amount of polymer deposited on the fluoropolymer surface.

Next, the contact angle of capillaries coated with gelatine was determined. A 10 bore microcapillary film fabricated from FEP having mean internal capillary diameter of 206 $\mu$m was filled with a 0.2 % gelatine solution and incubated overnight. The residual gelatine solution was pumped out of the microcapillaries using a manual syringe. The microcapillaries where then subsequently washed and/or dried using gaseous nitrogen. The contact angle of washed and dried microcapillaries was $\theta$ = 87$\pm$8.8 degrees, and of dried (unwashed) microcapillaries was $\theta$ = 74$\pm$12.0 degrees. The mean standard deviation between 3 consecutives contact angle measurements on the same coated microcapillaries was 1.60, demonstrating that the coating is stable and did not dissolve during the measurements in water.

The contact angle of capillaries coated with hydroxylpropylmethyl-cellulose (HPMC) was also measured. A 6% HPMC solution was prepared by dispersing the HPMC in hot deionised water (80°C) to prevent clumping. After the mixture cooled down, a clear solution was obtained. The solution was then injected manually into a 10 bore microcapillary film fabricated from FEP having mean internal capillary diameter of 206 $\mu$m and incubated overnight. The microcapillary film was then dried using gaseous nitrogen. The mean equilibrium contact angle of the HPMC coated capillaries was $\theta$ = 78$\pm$2.9 degrees.

In order to determine the contact angle of capillaries coated with a PVA-dextrin blend, equal volumes of 2 % PVA (Mwt 146,000-186,000) solution and a 1% dextrin solution were pre-mixed and injected into a 10 bore microcapillary film fabricated from FEP having mean internal capillary diameter of 206 $\mu$m and incubated overnight, after which the coated microcapillaries were dried with gaseous nitrogen. The mean equilibrium contact angle measured was $\theta$ = 67$\pm$8.0 degrees.

The contact angle of capillaries coated with a gelatine-PVA was measured as follows. First, individual stock solutions of both gelatine and PVA (Mwt of 146,000-186,000) were prepared at a concentration of 0.2 mg/mL and 2 mg/mL, respectively. Equal volumes of the stock solutions were then mixed to produce the gelatine-PVA (1:10 w/w) coating solution. A 10 bore microcapillary film fabricated from FEP having mean internal capillary diameter of 206 $\mu$m. The microcapillary film was then filled with the coating solution and incubated overnight. Finally, the solution gelatine was aspirated from the microcapillaries and the capillaries dried using gaseous nitrogen. The measured mean equilibrium contact angle was $\theta$ = 80$\pm$4.6 degrees.

The contact angle of capillaries coated with chitosan-based coatings was further determined. Three different procedures for chitosan-based coatings were tested, which explored the chemical properties of this polysaccharide.

For a low-chitosan concentration coating, a solution of 0.2% chitosan in 1% acetic acid was allowed to recirculate overnight at 0.5 mL/min through a 10 bore microcapillary film fabricated from FEP having mean internal capillary diameter of 206 $\mu$m. The microcapillaries were then washed with DI water at pH 7.0 and dried with gaseous nitrogen.

A high chitosan concentration coating was based on the method of Lin et al. (2005) for the coating of PE tubing. A 10 bore microcapillary film fabricated from FEP having mean internal diameter of 206 $\mu$m was filled with a solution of 2% chitosan in 1% acetic acid and then sealed and incubated for 2 hours. The solution was then replaced by methanol and incubated for further 30 minutes. After removal of methanol, the microcapillary film was dried at 40° C for 2h and dried with gaseous nitrogen. Finally, the microcapillary film was neutralised using a 5 wt % NaOH solution for 10 min and dried again at 40°C for 2 h.

A cross-linked chitosan coating was adapted from the method presented by Huang et al. (2011) for coating capillary columns. A 10 bore microcapillary film fabricated from FEP having mean internal capillary diameter of 206 $\mu$m was filled with a solution of 1% chitosan in 1% acetic acid and then sealed and incubated for 2 hours. The content of the capillaries was then substituted with a 1 mg/mL glutaraldehyde solution, and cross-linking allowed to proceed for 15 minutes. The microcapillaries were then dried using gaseous nitrogen. The previous steps were repeated for further two times in order to give a total of three layers of cross-linked polysaccharide. The measured mean equilibrium contact angle was $\theta$ = 80$\pm$9.8 degrees.

Finally, the contact angle of capillaries coated with alginate-based coatings was determined. For the alginate/calcium coating (alginate/CaSO$_4$), a 10 bore microcapillary film fabricated from FEP having mean internal diameter

of 206 μm was incubated overnight with a 1% Sodium Alginate solution. The alginate solution was then carefully flushed out using a manual syringe. A calcium sulphate solution (1%) was then injected into the microcapillaries for cross-linking the adsorbed alginate, and incubated for 30 minutes after which the microcapillaries were dried with gaseous nitrogen. The measured mean equilibrium contact angle was $\theta$ = 88±4.6 degrees.

For the alginate-chitosan blend coating: a 10 bore microcapillary film fabricated from FEP having mean internal capillary diameter of 206 μm was loaded with equal volumes of a stock solution (0.25%) of sodium alginate vigorously pre-mixed for 20 min with a stock solution of chitosan. The microcapillaries were incubated overnight and then dried using gaseous nitrogen. The measured mean equilibrium contact angle was $\theta$ = 93±4.5 degrees.

The alginate/chitosan multilayer coating (Alg/Ch/Alg/Ch) was based on the work of Carneiro-da-Cunha et *al.* (2010) for the fabrication of chitosan/alginate nanolayered PET film. A 10 bore microcapillary film fabricated from FEP having mean internal capillary diameter of 206 μm was filled with a 0.2 % sodium alginate solution (pH 7) and incubated overnight. The microcapillary film was then emptied and filled with a 0.2 % chitosan solution in 1% acetic acid (pH 3) and incubated for 4 hours, after which the solution was manually removed. This was repeated keeping each solution inside the microcapillary film for at least 2 hours. The coated microcapillaries were then dried using gaseous nitrogen. The measured mean equilibrium contact angle was $\theta$ = 93±2.6 degrees.

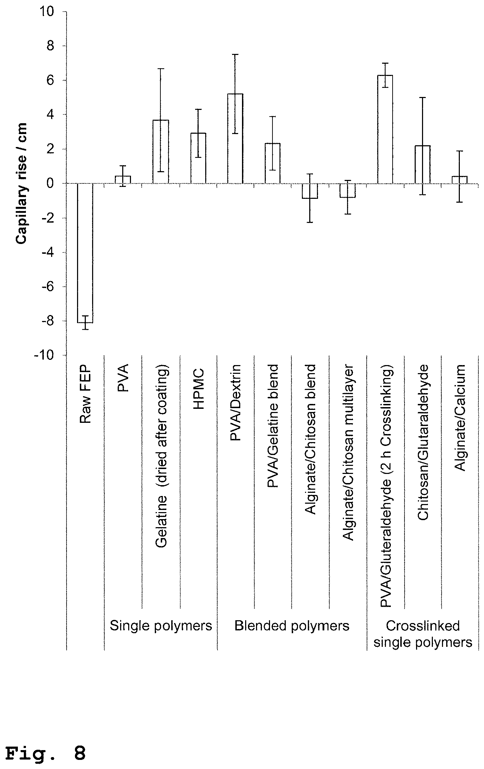

Fig. 8 shows the capillary rise (in cm) in FEP microcapillaries coated with some of the different hydrophilic layers described above. As can be seen from this figure, only hydrophilic layers composed of low molecular weight polyvinylalcohol (PVA), cross-linked low molecular weight polyvinylalcohol (PVA), gelatine, hydroxypropylmethylcellulose (HPMC), PVA and dextrin, PVA and gelatine, cross-linked chitosan, or alginate and calcium, were capable of promoting capillary rise in the coated capillaries. High molecular weight PVA was not tested in this experiment. Even a small capillary rise may, for example, be sufficient where the capillaries of an assay device are short. Alternatively, capillaries capable of promoting only a small capillary rise can be incubated horizontally, thereby allowing the sample fluid to rise further in the capillary by capillary action. As a further alternative, the top (rather than the bottom) of the capillary may brought into contact with a sample fluid, thereby allowing the sample to flow down the capillary by capillary action. None of these options are available where the capillaries are hydrophobic.

For all subsequent experiments, capillaries coated with cross-linked low molecular weight PVA were used.

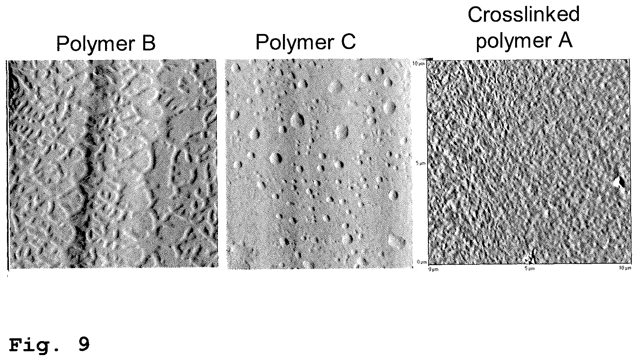

Fig. 9 further shows that even coating of the capillary wall by the hydrophilic layer improves capillary rise of samples in the capillary. The most even coating was achieved with cross-linked PVA (PVA cross-linked with glutaraldehyde) ("polymer A" in Fig. 9). Thus, preferably, the hydrophilic layer comprises or consists of cross-linked PVA, such as PVA cross-linked with glutaraldehyde.

Next the effect of retaining assay reagents, such as antibodies, in the hydrophilic layer on the contact angle of the coating was determined. Specifically, contact angles were measured following incubation of coated microcapillaries with an antibody. 10 bore FEP microcapillary films pre-coated with cross-linked PVA, gelatine, HPCM, or PVA-dextrin, as described above was incubated for 2h at room temperature with an IgM antibody solution (1 mg/mL). The microcapillaries were then dried using gaseous nitrogen and the equilibrium contact angle measured. The mean equilibrium contact angle remained unchanged in the case of the cross-linked PVA ($\theta$=60±2.8 degrees), and gelatine ($\theta$=70±1.3 degrees) coatings, increased slightly in the case of the HPCM coated capillaries ($\theta$=84±1.9 degrees) and was reduced slightly in the PVA-dextrin coated capillaries ($\theta$=58±2.3 degrees). However, in all cases the equilibrium contact angle remained significantly improved compared with uncoated FEP microcapillaries, indicating that reagents can be retained in the hydrophilic layers while maintaining the advantageous properties of the coated capillaries with respect to promoting capillary rise of a liquid sample introduced to the capillary.

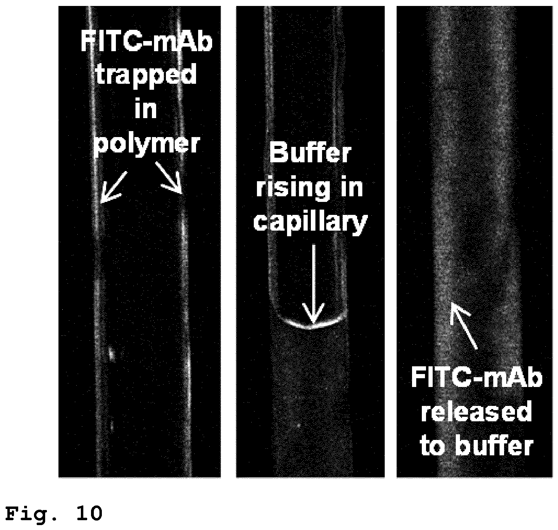

In order to demonstrate the possibility of loading assay reagents in dried coated capillaries and rapidly releasing reagents during sample uptake by capillary action, a 10 bore FEP microcapillary film internally coated with cross-linked PVA was loaded with 1 mg/ml fluorescein isothiocyanate (FITC)-labelled IgM and incubated for 2 hours and then dried using gaseous nitrogen. One end of the capillary was then immersed in 20 mM PBS pH 7.4 buffer and fluorescence monitored using a confocal fluorescence microscope using excitation wavelength of 488 nm. Figure 10 shows (from left to right) a dried and loaded capillary, a loaded capillary partially filled with buffer, and a loaded capillary fully filled with buffer. The sharp interface in the partially filled capillaries showed a high concentration of antibody being transported during liquid rising through the capillary by capillary action. In both the partially and the fully filled capillary, diffusion of the antibody out of the hydrophilic layer into the buffer can be seen, demonstrating that the antibody is rapidly released from the hydrophilic layer into the sample fluid.

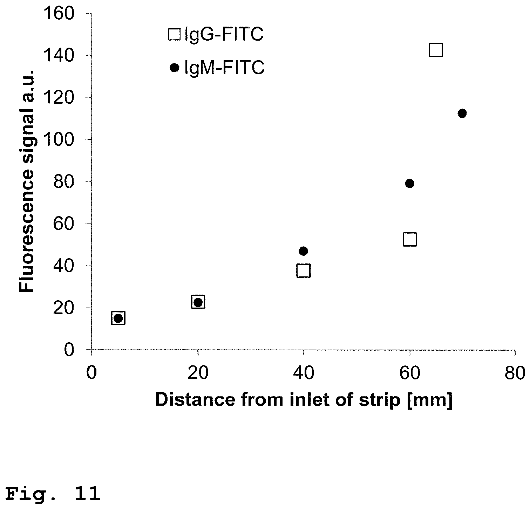

The effect of molecular weight on reagent release was determined as follows. A 10 bore FEP microcapillary film coated with cross-linked PVA was loaded with a 1 mg/ml IgG-FITC or IgM-FITC solution and incubated for 2 hours. Excess liquid was aspirated manually using a plastic syringe. One end of the strip was then immersed in 20 mM PBS pH 7.4 buffer and fluorescence monitored using a confocal fluorescence microscope using an excitation wavelength of 488 nm, after the liquid-air interface reached the equilibrium point. The concentration of fluorescently labelled antibody was measured along the height of the buffer-filled capillary. The results are shown in Fig. 11. This figure demonstrates that the higher molecular weight IgM antibody was released from the hydrophilic layer more slowly than the lower molecular weight IgG antibody. The IgG antibody showed a peak in concentration at the air-liquid interface. However, these results demonstrate that even very high molecular weight assay reagents, such as IgM, can be retained using a hydrophilic coating such as cross-linked PVA.

Reagent loading and release from the hydrophilic layer was quantified using a fluorescent dye and colorimetric enzyme substrates. For this purpose, an FEP microcapillary film was coated with cross-linked PVA and loaded with two assay reagents, the enzymatic substrate ortho-nitrophenyl-p-galactoside (ONPG) which produces a yellow colour in the presence of beta-galactosidase, and the fluorescent dye fluorescein. Loading was performed by incubation with a water or dimethylformamide (DMF)-based reagent solution for between 5 minutes and 2h, and subsequent removal of the loading solution using a syringe, followed by air drying of the capillaries by repeated aspiration of air through the film using a syringe. Following loading of the capillaries, the amount of reagent loaded was quantified using three different methods.

Firstly, 60mm long strips loaded for 1 hour with ONPG dissolved in water at concentrations from 0.5 to 5.0 mg/ml or dissolved in DMF at a concentration of 150 mg/ml were tested by dipping into water which resulted in water uptake into the capillaries. The water taken up was then removed using a syringe and ONPG concentration measured by enzymatic endpoint analysis in a microwell plate well through the addition of an excess of beta-galactosidase enzyme and monitoring absorbance at 420nm using a microwell plate reader. Released concentrations were determined by comparison of absorbance with a calibration curve of known concentrations of ONPG. The results are shown in Table 1.

Table 1

| Compound | Loading concentration (mg/ml) | | Released Concentration (mg/ml) |
|---|---|---|---|
| ONPG | In Water | 0.0 | 0 |
| | | 0.5 | 0.07 |
| | | 1.0 | 0.01 |
| | | 5.0 | 0.06 |
| | In DMF | 150.0 | 4.67 |

Secondly, the concentration of loaded ONPG was determined inside the capillary film by dipping an FEP microcapillary film coated with cross-linked PVA, and loaded for 5 minutes with 150 mg/ml ONPG dissolved in DMF, into aqueous solutions of beta-galactosidase enzyme (at 0.2U/$\mu$L). The solution was drawn up into test strips, and incubated for 1 hour to allow complete enzymatic conversion of the loaded ONPG. Subsequently, solution was removed from MCF test strips using a syringe and absorbance read using a microplate reader at 420nm, and absorbance compared to a calibration curve of known concentrations of enzymatically converted ONPG. In this test, an average concentration of 3.4mg/ml ONPG was found to be loaded within these ONPG loaded test strips.

Thirdly, an FEP microcapillary film coated with cross-linked PVA was loaded with fluorescent dye through a 5 minute incubation with 100 $\mu$g/mL of fluorescein dissolved in water followed by removal of the solution and air drying by repeated passing air through the strips with a syringe. Then, 80mm long loaded microcapillary films were tested by dipping into water, and the concentration of loaded fluorescein was measured within the test strip by imaging strips illuminated with 450nm blue light and imaging green fluorescence using a digital camera through a longpass filter with cut-off of 500nm. The fluorescence intensity within test strips dipped in water was compared with a calibration curve of fluorescence intensity observed in FEP microcapillary films filled with known concentrations of fluorescein. The concentration of fluorescein in the dipped test strips varied along the length of the test strip, indicating very rapid release of reagent such that at the inlet where water entered the strip, the concentration of fluorescein was too low to be measured using the fluorescence imaging system used. In contrast, at the top of the test strip, a high concentration of fluorescein was seen, with a gradient of fluorescein observed (Table 2). These results agree with the release pattern seen with IgM and IgG reported above.

Table 2

| | Test strip 1 | | | | Test strip 2 |
|---|---|---|---|---|---|
| Distance from inlet: | 30mm | 40mm | 60mm | 70mm | 70mm |
| capillary number | Fluorescein concentration $\mu$g/ml | | | | |
| 1 | 0.1 | 0.4 | 1.9 | 2.2 | 1.1 |

(continued)

|  | Test strip 1 | | | | Test strip 2 |
| --- | --- | --- | --- | --- | --- |
| Distance from inlet: | 30mm | 40mm | 60mm | 70mm | 70mm |
| capillary number | Fluorescein concentration $\mu$g/ml | | | | |
| 2 | 0.2 | 0.3 | 1.0 | 1.2 | 1.1 |
| 3 | 0.1 | 0.4 | 1.0 | 1.2 | 1.1 |
| 4 | 0.1 | 0.4 | 1.0 | 1.2 | 1.3 |
| 5 | 0.1 | 0.3 | 1.0 | 1.3 | 1.1 |
| 6 | 0.2 | 0.4 | 1.1 | 1.2 | 1.1 |
| 7 | 0.2 | 0.8 | 1.4 | 1.1 | 1.1 |
| 8 | 0.3 | 0.5 | 1.0 | 1.0 | 1.4 |
| 9 | 0.2 | 0.6 | 1.2 | 1.2 | 1.1 |
| 10 | 0.1 | 0.4 | 1.4 | 2.4 | 1.0 |

These above results show that different classes of reagents with differing physicochemical properties, such as differing molecular weights, can be retained in the capillaries using hydrophilic layers, such as cross-linked PVA, with similar efficiency. This demonstrates that there is no specific physicochemical requirement for assay reagents to be retained.

Table 2 further shows that an assay reagent retained in the hydrophilic layer is released evenly in all capillaries of a single test strip. Secondly, the reagent mixes rapidly with the sample fluid and forms a gradient of reagent in the sample fluid, as also seen in Fig. 11. Table 2 also demonstrates that the concentration of assay reagent released from the hydrophilic layer is typically 1% of the concentration of the loading solution.

To further demonstrate that assay reagents of different types can be retained at the hydrophilic layer, glucose was loaded into test strips to allow detection of microorganisms capable of fermenting glucose in a test sample. As before, FEP microcapillary film coated with cross-linked PVA was loaded using a loading solution 700mg/ml glucose in water. After removal of loading solution and drying of the capillaries, test strips were cut and dipped into water. The water taken up into the test strips was removed using a syringe and the concentration of glucose released into the water was then measured using a commercially available enzymatic glucose assay utilising hexokinase and glucose-6-phosphate dehydrogenase. The concentration of glucose released was 2.9mg/ml, thus demonstrating that glucose can be retained at a hydrophilic layer, such as cross-linked PVA.

The effect of viscosity of the loading solution on the ability of the loading solution to load a hydrophilic layer with assay reagent was also investigated. In this example, a 100$\mu$g/ml solution of fluorescein dissolved in water alone or water plus glycerol at a concentration of 0%v/v, 10%v/v, 50%v/v or 80%v/v was introduced into a FEP microcapillary film coated with cross-linked PVA. The viscosity of these glycerol solutions was 1.31 mPa·s, 6.00 mPa·s, and 60.1 mPa·s, respectively, when measured at 20°C. After a 5 minute incubation, the loading solution was removed and the capillaries dried by removal of the loading solution by suction using a syringe. The loaded FEP film was cut into 10cm long strips and dipped into water which was rapidly drawn up into the capillaries. The concentration of fluorescein released into the water was measured within the test strip by imaging strips illuminated with 450nm blue light and imaging green fluorescence using a digital camera through a longpass filter with cut-off of 500nm. By comparing the green fluorescence intensity with control strips filled with known concentrations of fluorescein, the concentration of dye at 4cm from the end of the capillaries dipped into the water was found to depend on the concentration of glycerol in the solution used to load capillaries with fluorescein as follows: as the concentration of glycerol in loading solution was increased from 0% to 10%, 50% and 80% v/v, the amount of fluorescein released increased from 1.9$\mu$g/ml, to 4.5$\mu$g/ml, 6.3$\mu$g/ml to 14.4pg/ml respectively. These results demonstrate that increasing the viscosity of the loading solution increases the amount of assay reagent retained at the hydrophilic layer. This suggests that a film of the assay reagent is deposited on the hydrophilic layer. It is thought that increasing the viscosity of the loading solution makes this reagent layer thicker by increasing capillary number, Ca, resulting in higher retention of reagent.

The extruded microcapillary film (MCF) coated internally with a hydrophilic layer can thus be used to form the basis of a new platform for assays, such as blood typing and bacterial identification assays, providing a simple and cost effective method therefore. The key aspects of the preferred embodiments of this invention involve:

1. Coating each microcapillary internally with a hydrophilic layer. This can be done by adsorbing polymers such as polyvinylalcohol onto the inner surface of the microcapillary film (MCF), optionally followed by cross-linking. For example, capillaries can be coated with 20 mg/ml of PVA solution in water, and then fluid aspirated leaving a thin-film at the surface of the capillary bore. The polymer coats the plastic surface by passive adsorption.

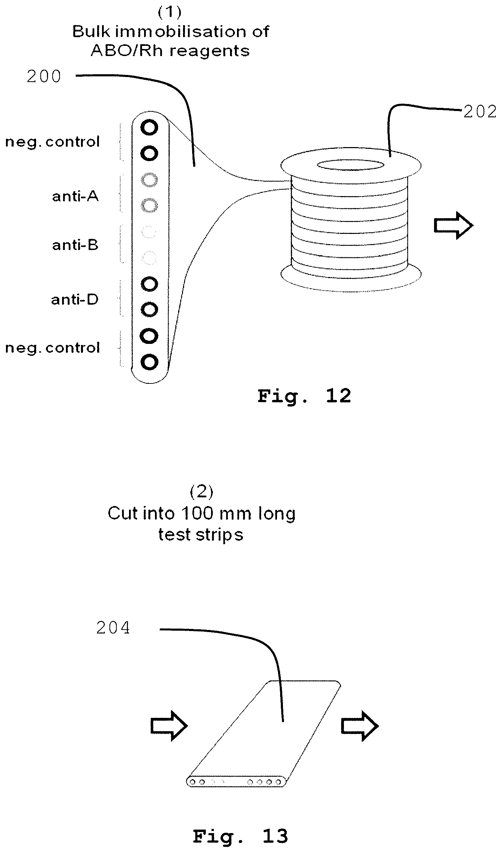

2. Loading each individual microcapillary with a specific assay reagent. This can be done by injecting appropriate solutions down respective channels of an MCF that may be several metres in length. The solutions is then left to rest within the capillary at a constant temperature for a few minutes (Fig. 12). The solution is then dried by allowing an inert gas, such as nitrogen, to gently flow through the capillaries or by mopping up the solution using an absorbent material such as paper tissue

2. The MCF can then be cut into lengths of, e.g., 1 cm (Fig. 13). This represents a very effective manufacturing route for assay devices.

3. Operation is effected by allowing a sample fluid to rise in the channels of the MCF by capillary action. The assay reagent(s) embedded in the hydrophilic layer then diffuse out and react with any substance of interest present in the sample fluid.

4. Detection can be by naked eye or automated.

Extruded FEP MCF is available from Lamina Dielectrics, Ltd., Daux Road, Billingshurst, West Sussex RH14 9SJ, United Kingdom.

Figs. 12-14 illustrate a method of manufacturing an assay device according to an embodiment of the present invention.

In Fig. 12, a length of microcapillary film 200 is provided on a reel 202. The capillaries are coated with a hydrophilic layer. The film is manufactured from FEP, as discussed above. The total length of the microcapillary film can be, for example, at least 1 m. Longer lengths, e.g. up to 5m, 10 m, 20 m, or longer still can be manufactured via the extrusion process discussed above.

Subsequently, it is possible to load each microcapillary with an assay reagent, such as an antibody. This may be done, for example, using capillary action or a syringe and needle. Conveniently, the loading step can be carried out whilst the MCF remains on a reel. Where it is wanted to load each microcapillary identically, this can be carried out by dipping one end of the MCF into a single loading solution, this loading solution being taken up into each capillary bore by capillary action or aspirated into each microcapillary bore using a single aspirator (not shown) at the opposite end of the MCF. Where it is wanted to load one or more microcapillaries differently, the microcapillaries can be separated at one end of the MCF, either into individual microcapillaries or into set of microcapillaries.

Separation may be achieved by cutting the film between the capillaries. Separating the microcapillaries in this way, facilitates contacting each microcapillary, or set of microcapillaries, with a different loading solution. This is useful where each microcapillary or set of microcapillaries is to be loaded differently. The loading solution can be taken up into each capillary bore by capillary action or aspirated into each microcapillary bore or set of microcapillary bores, using a single aspirator. Alternatively, a separate aspirator can be used for each capillary or set of capillaries. The aspirator may, for example, be a pipette or a syringe.

As shown in Fig. 12, duplicates of microcapillary bores can be loaded with the same assay reagent, e.g. antibody, to provide measurement redundancy in the device.

Subsequently, the loaded microcapillary is cut to a desired length (e.g. 5-50 mm), as shown in Fig. 13, to form an assay device 204. As will be understood, the long reel of MCF can be used to form very many assay devices. Therefore only a coating and a single loading step is required, even though very many assay devices are produced.

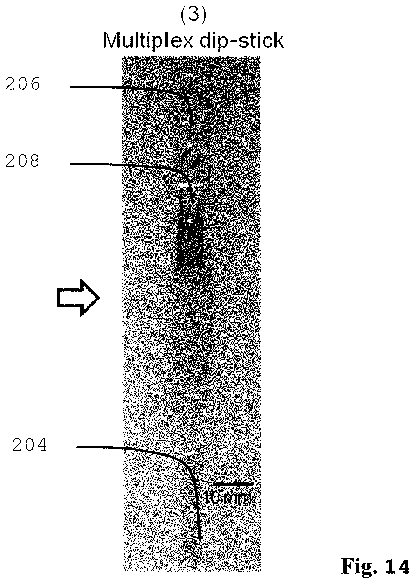

Next, as shown in Fig. 14, the assay device 204 can be placed in a plastic case 206 and used to carry out a blood typing assay by immersing one of the ends of the test strip into a small blood drop or other liquid sample. When the free end of the device 204 is dipped into a blood sample, capillary action causes the blood to be drawn into and rise in the capillary bores. The observation window 208 allows agglutination to be optically detected near $h_{max}$.

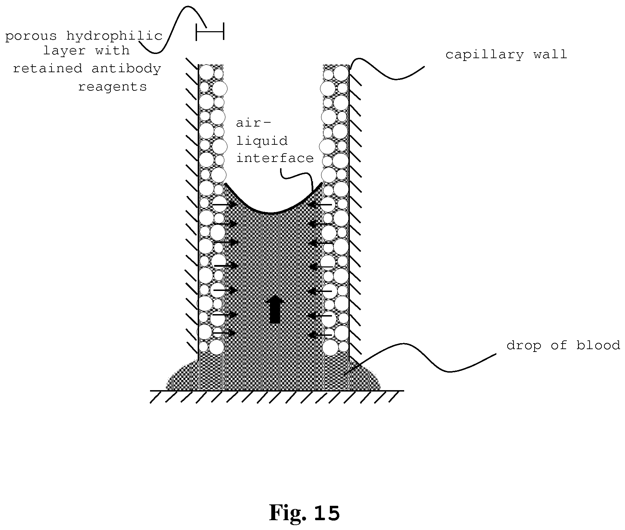

As shown in Fig. 15 the antibodies retained in the hydrophilic coating of the MCF capillaries are quickly released. The hydrophilic coating is preferably cross-linked with cross-linking agents known to those of the art, for reducing the contact angle of the sample with the wall and increasing the velocity of the blood rising in the capillaries.

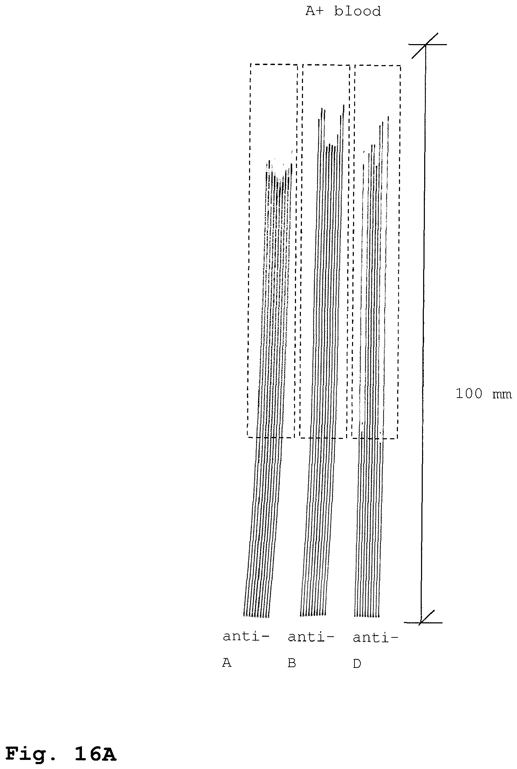

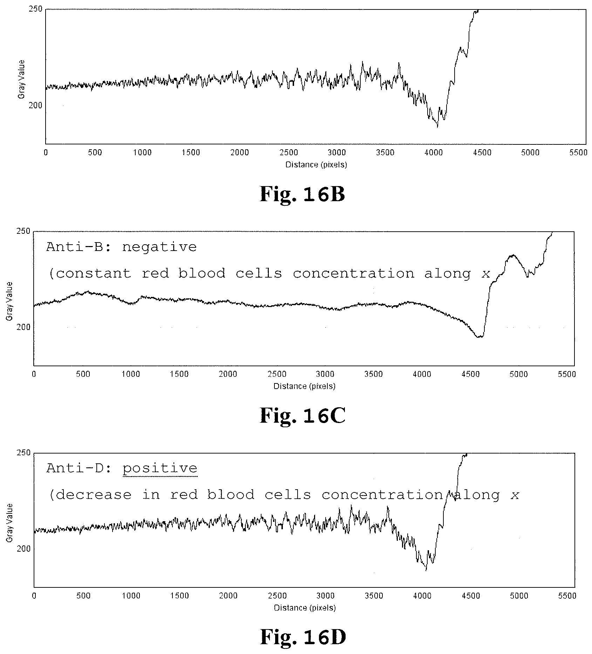

The experiments shown in Figs. 16-19 were all conducted in a MCF-FEP coated with PVA as the hydrophilic layer. The thickness of the hydrophilic layer was 1-2 $\mu$m. The molecular weight of the PVA used for coating the MCF-FEP capillary bores was 13,000 to 23,000 g/mol.

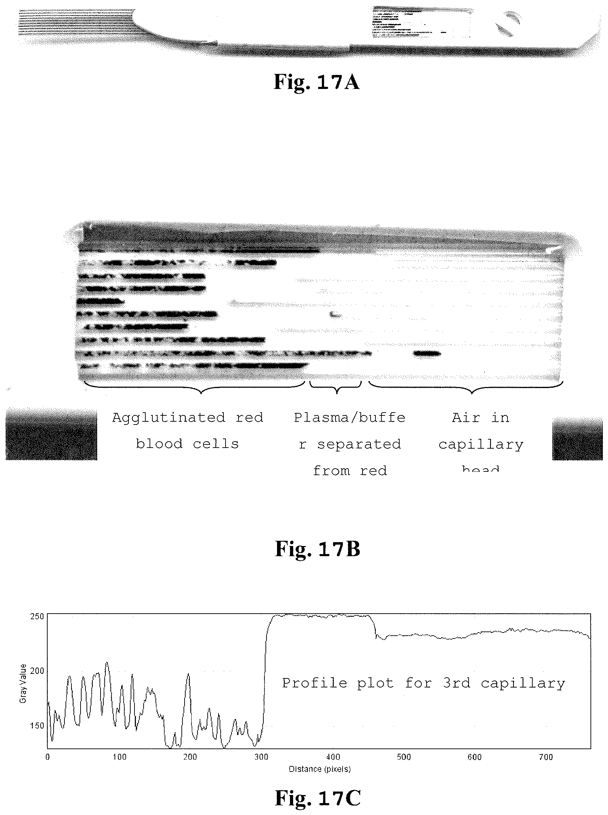

Where the red blood cells present in the blood sample do not have the antigen to which the antibody retained in the hydrophilic layer specifically binds on their surface, it was found that the blood flowed into and rose in the capillary bore in the normal way. However, where red blood cells present in the blood sample had the antigen to which the antibody retained in the hydrophilic layer specifically binds on their surface, an agglutination reaction occurred. This resulted in the agglutination of red blood cells in the capillary with a visible separation of the red blood cells and the blood serum along the capillary (Figs. 16A-D and 17A-C), In addition the agglutinated blood sample reached a lower height in the capillary bore then the agglutinated red blood cells (Fig. 16C). In the absence of agglutination, the blood can reach a maximum height, $h_{max}$ in the capillaries dictated by the ratio of the surface tension to resistance (viscous) forces, which according to Young-Laplace equation can be determined from the equilibrium contact angle and the surface tension of whole blood with air.

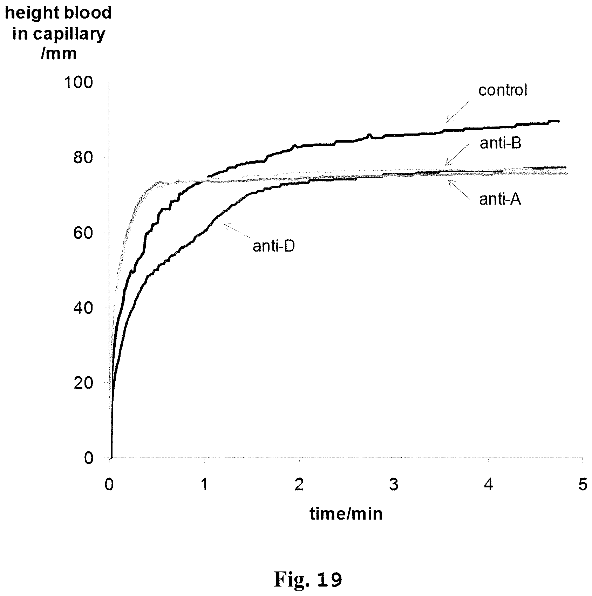

From both the optical and height response of the blood sample in each capillary an agglutination test can then be detected after only a few seconds, preferably after 120 seconds and ideally after 30 seconds (Fig. 19) in different ways.

For example, one can measure a greyscale intensity across the microcapillary array near $h_{max}$ (Fig. 18A). For the capillaries where the blood did not agglutinate (therefore capable of reaching the maximum height in the capillary, i.e. $h_{max}$) the plot would reveal a strong signal corresponding to strong light absorption, whereas for the capillaries where the red blood cells have agglutinated upon the release of the complementary antibody the plot would show no signal compared to background because of the absence of red blood cells in the area scanned.

Alternatively, a short section of the capillary, preferably 5 mm or less in length can be imaged across the microcapillary array at a reference distance, e.g. $h_{max}/2$ (Fig. 18B) and the result of the agglutination optically detected by a skilled person, as agglutinated blood present as patches of red blood cells e.g. Figs. 16B and 18E), whereas non-agglutinated presents as a very homogenous colour intensity plot along the imaging section (Figs. 16C and 18G).

As a further alternative, agglutination can be detected by plotting the greyscale along each individual capillary in the scanned area, with the agglutinated red blood cells giving high signal variability. For example, the capillaries can be imaged by illuminating the background of the capillary film with an LED and scanning top of the capillary film with a CCD detector, such as a CCD technology flatbed scanner in transmittance mode at a minimum resolution of 1,200 dpi, as in the case of Figs. 16A-18L. RGB images of the capillaries were acquired using the CCD technology flatbed scanner and then split into three different channels, red, green and blue, and converted to 8 bits using image analysis software ImageJ, a free program provided by the National Institute of Health, USA. That was the case in Fig. 16A and Fig. 18A. Using the same 8 bit images, a greyscale profile was plotted along each individual capillary. A greyscale value of 255 represents white colour (100% light transmittance), whereas a greyscale value of 0 represents black (0% light transmittance). Non-agglutinated blood present as a smooth greyscale profile (see e.g. Fig. 16C), while a variable greyscale profile indicates the presence of agglutinated blood in the capillary (see e.g. Fig. 16D).

Preferably the presence of agglutination is determined using a device incorporating a CCD detector, which is also capable of image analysis and the user is presented with a yes/no answer regarding the presence of agglutinated blood in each capillary.

The flat surfaces of the MCF material coupled with a close matching in refractive index between the wall material and the liquid sample allow measuring the concentration of red blood cells along and across each individual capillary using cheap optical detection systems such as (but not limited) to CCD cameras, digital microscopes, digital cameras, smartphone cameras or flatbed scanners. Thus, capillaries can be individually cross-interrogated using cost-effective optical detectors. Close matching in refractive index between the wall material and the liquid sample also improves ease of detection of agglutinated blood samples in the capillaries using the naked-eye.

In addition to blood typing, the assay devices of the invention can be used for other applications, including the identification of bacteria present in a test sample.

For example, *E. coli* can be identified by its ability to inducibly express beta-galactosidase activity in the presence of a substrate for this enzyme. In contrast, *Salmonella,* which are otherwise similar gram negative bacteria to *E. coli,* cannot express beta-galactosidase. The ability to convert beta-galactosidase substrates such as ortho-nitrophenyl-p-galacoside (ONPG) is therefore widely used as an identification test to distinguish *E. coli* from *Salmonella.* When *E. coli* are cultured overnight in LB broth, low levels of beta-galactosidase activity are observed, however after culture in the presence of a substrate such as ONPG, lactose, Isopropyl $\beta$-D-1-thiogalactopyranoside (IPTG), or galactose, high levels of beta-galactosidase enzyme are expressed.

A 10 bore FEP microcapillary film was internally coated with cross-linked PVA. Test microcapillary film was prepared by first loading the capillaries with ONPG by filling the capillaries with a 150 mg.mL$^{-1}$ ONPG substrate solution in DMF and incubating for 5 minutes. The substrate solution was then removed and the microcapillary film air dried using a syringe to pass air repeatedly through the capillaries. Seven test strips were cut from this ONPG loaded microcapillary film and dipped into one of 7 different aqueous samples. These comprised water (1); sterile Luria-Bertani lysogeny broth (LB) media (2); a 0.1 U/μl solution of β-galactosidase enzyme (3); an overnight culture of *E. coli* (ATCC accession number: 25922) suspended in LB (4); an overnight culture of *Salmonella typhimurium* (strain SL3261) suspended in LB (5); an overnight culture of *E. coli* suspended in a phosphate buffer saline PBS (6); an overnight culture of *S. typhimurium* suspended in PBS (7). Negative control test strips consisting of PVA coated cross-linked PVA but not loaded with ONPG were dipped into the same samples (referred hereafter as no substrate). As a comparison, similar assays were also performed in 96 well-plates with ONPG substrate solution (final concentration 1mg.ml$^{-1}$) added to the different samples in a final volume of 100 μl.

In the control experiments performed in 96-well plates, no yellow colour (which would indicate enzymatic conversion of ONPG) was observed in either bacterial suspension. After overnight incubation to allow induction of beta-galactosidase activity, wells containing *E. coli* but not *S. typhimurium* suspensions turned yellow, indicating induction of beta-galactosidase activity and thereby allowing identification of *E. coli.*

When the ONPG loaded cross-linked PVA coated MCF test strips were used, a similar pattern of yellow colour production was observed. When dipped into samples, test strips rapidly sucked up aqueous samples into all capillaries, as expected. After 5 minutes incubation, strips dipped into sample (3) turned yellow, indicating a rapid reaction between ONPG and the positive control beta-galactosidase enzyme solution. In contrast, no other test strip changed colour, indicating as expected that neither LB broth, nor bacterial suspensions, contained beta-galactosidase enzyme. However, test strips dipped into overnight cultures of *E. coli* (suspensions (4) and (6)), turned dark yellow, indicating the presence of beta-galactosidase enzyme. In contrast, test strips dipped into LB (2) or suspensions of *S. typhimurium* (5) and (7) did not change colour, indicating that neither the bacterial growth medium, nor the buffer, contained beta-galactosidase. As expected, no yellow colour was observed in any of the control test strips coated with cross-linked PVA but not loaded with ONPG. These results are summarised in Table 3.

Table 3 - Qualitative detection of *E. coli* in FEP microcapillaries coated with cross-linked PVA and loaded with ONPG

|  | Incubation time | | | |
|---|---|---|---|---|
|  | ONPG loaded capillaries | | Non-loaded capillaries (control) | |
| Sample | 5 min | Overnight | 5 min | Overnight |
| (1) Water | - | - | - | - |
| (2) LB broth | - | - | - | - |
| (3) beta-galactosidase solution | + | + | - | - |
| (4) *E. coli* in LB broth | - | + | - | - |
| (5) *S. typhimurium* in LB broth | - | - | - | - |
| (6) *E. coli* in PBS | - | + | - | - |
| (7) *S. typhimurium* in PBS | - | - | - | - |

A further experiment was conducted to demonstrate rapid detection of bacterial enzymatic activity. Overnight cultures of *E. coli* and *S. typhimurium* were diluted 1:100 in LB broth with the inclusion of the beta-galactosidase inducer IPTG (final concentration 1mM) for 3 hours, and then tested using OPNG loaded FEP microcapillary test strips coated with cross-linked PVA. Under these conditions, some bacterial strains including *E. coli* are able to produce high levels of beta-galactosidase, whereas others including *S. typhimurium* do not.

As above, test strips were loaded with ONPG and tested by dipping into samples of either no bacteria, IPTG-cultured *E. coli,* or IPTG-cultured *S. typhimurium.* The colour of the ONPG-loaded test strips dipped into these three samples was examined after 1 hour incubation at 37 degrees C. In the test strips dipped into samples of IPTG-cultured *E. coli,* a yellow colour was observed, indicating the presence of high levels of beta-galactosidase. In contrast, in the test strips dipped into samples of IPTG-induced *S. typhimurium* or no bacteria, no yellow colour was observed, and the test strips remained colourless, indicating that these bacteria were unable to produce beta-galactosidase. This demonstrates that ONPG loaded test strips are useful for rapidly distinguishing very similar bacteria that have different beta-galactosidase enzymatic capacity.

The ability of bacteria to ferment specific sugars is also frequently used to distinguish similar species of bacteria. For example, *E. coli* are able to ferment lactose and glucose and produce acid when they are incubated in the presence of either of these sugars. In contrast, Salmonella such as *S. typhimurium* are only able to ferment glucose and produce acid in the presence of glucose, but are not able to ferment lactose and do not produce acid when incubated in the presence of lactose.

A 10 bore FEP microcapillary film was internally coated with cross-linked PVA. Test microcapillary film was prepared by first loading the capillaries with either glucose or lactose by filling the capillaries with a 1g/L solution in water and incubating for 5 minutes. The sugar solutions were then removed and the microcapillary film air dried using a syringe to pass air repeatedly through the capillaries. Three test strips were cut from each of the glucose or lactose loaded microcapillary film and dipped into one of 3 different aqueous samples. The first sample was a control sample that contained no bacteria. The second sample contained a suspension of *E. coli.* The third sample contained a suspension of *Salmonella* typhimurium. All three aqueous samples also contained Phenol Red Broth (Casein peptone 1g/L; NaCl 0.5g/L; Phenol Red 1g/L). The samples all started out with a pH of 7 and were bright red in colour. When dipped, each aqueous sample was rapidly drawn up into the test strips, and the microcapillaries were clearly bright red in colour due to the Phenol Red indicator dye in the sample. All test strips were then incubated for 1h at 37 degrees C. In the presence of either glucose or lactose, the strips dipped in the control sample without cells remained a bright red colour. As expected, both glucose and lactose loaded test strips dipped into samples with *E. coli* turned yellow indicating a reduction in pH and therefore demonstrating the presence of bacteria capable of fermenting both glucose and lactose in these samples. In contrast, only the glucose loaded test strip dipped into the sample containing *S. typhimurium* turned yellow, while the lactose loaded strips remained bright red, confirming the presence of a bacterial strain which is capable of fermenting glucose but not lactose.

To demonstrate that hydrophilic coatings other than cross-linked PVA can also be used for the retention of assay reagents, FEP microcapillary film was coated with the polymers HPMC (hydroxyl propyl methylcellulose) and gelatine by incubating FEP microcapillary films filled with a 0.2%w/v gelatine solution or a 0.6%w/v HPMC solution overnight. The polymer solutions were then removed from the microcapillary film and the film dried by repeatedly aspirating air through the film strip using a syringe. An FEP microcapillary film coated with cross-linked PVA was used as a control. 8 cm lengths of each polymer coated film were then loaded with ONPG reagent by dipping the film into a 150 g/L ONPG solution. After a 5 minute incubation, the ONPG solution was removed and capillaries were dried using a syringe. The test films were then dipped into water, and the amount of ONPG reagent released into the water measured by a colorimetric endpoint determination using beta-galactosidase enzyme. With the cross-linked PVA coated test strips, 7.8mg/ml ONPG was released, with gelatine coated test strips 7.2mg/ml ONPG was released, and with HPMC coated test strips 21.5mg/ml ONPG was released. These results demonstrates that a range of different hydrophilic coatings can be used to retain assay reagents in the capillaries, and that these assay reagents are further rapidly released into a liquid sample drawn up into the capillaries by capillary action.

Next it was tested if assay reagents can be immobilized at the hydrophilic layer. This is useful, for example, for quantitative ELISAs where the capture antibody needs to be immobilized on a surface.

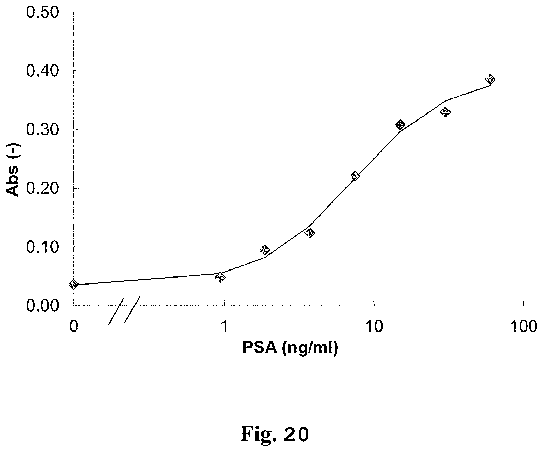

A 10 bore FEP microcapillary film was coated with low-molecular weight PVA and cross-linked with glutaraldehyde for 2h, as described for other examples above. A 40 μg/ml solution of a monoclonal antibody specific for human Prostate Specific Antigen (PSA) was then added to the capillaries and the antibody covalently bound to the hydrophilic layer by incubating sequentially in 5% v/v glutaraldehyde and then 1 mg/ml $NaBH_4$, and then blocked with 3% BSA. The microcapillary film was then cut into a number of 8 cm long test strips, and interfaced to a number of microwells via push-fit seals. The device was placed in the vertical position, and immunoassay solutions sequentially loaded into the well. Because of the hydrophilic nature of the coated capillaries, the solutions ran by gravity until no solution was left in the wells. The sequence of solutions in the sandwich ELISA assay involved recombinant protein with the standards for 5 minutes, 1 μg/ml biotinylated-labelled antibody for 5 minutes, 1 μg/ml high-sensitivity streptavidin-HRP enzyme conjugate for 5 minutes. The strips were then washed 4 times with PBS-Tween, after which 4 mg/ml of chromogenic substrate OPD was added. The volume of reagent added to the wells on each step was 200 microliters. Binding of the detection antibody to PSA bound by the capture antibody was determined from the grey scale of the blue channel split from RGB image of the microcapillary film captured with a flatbed scanner for 2 min incubation of OPD substrate. Figure 20 shows that as the amount of recombinant PSA protein in the sample increased, the absolute Absorbance signal increased, demonstrating both that the capture antibody was successfully immobilized at the hydrophilic layer, and making quantitative detection of antigen possible.

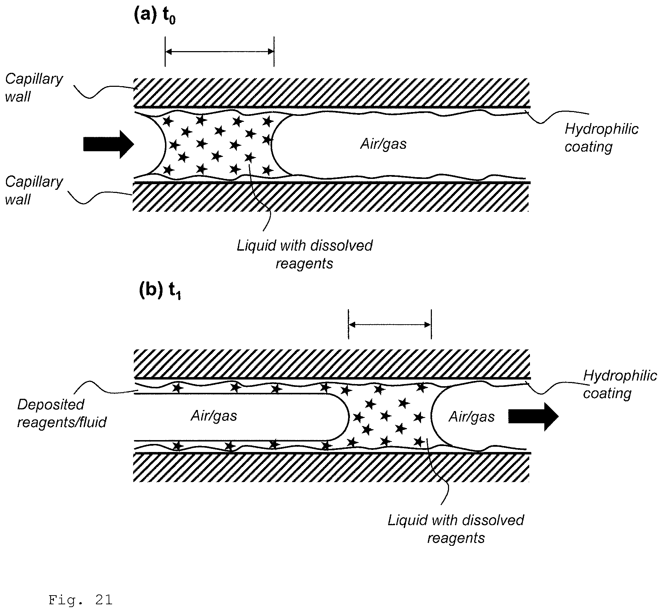

Two methods of including a reagent into the hydrophilic coating layer were tested. First, a 30mm long fluid slug containing assay reagent was manually injected into a 30 cm long, 200 micron i.d. FEP Teflon microcapillary coated with cross-linked HMW PVA, as shown in Fig. 21A. The fluid slug was then aspirated from the other end of the capillary using a vacuum pump as shown in Fig. 21B.

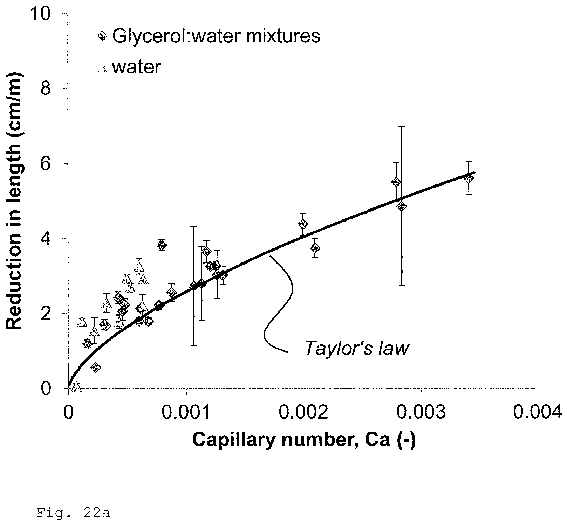

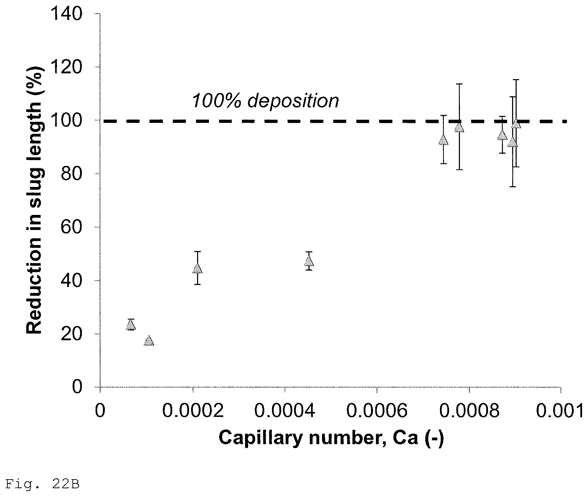

The time for the slug to travel the 30cm distance and the corresponding final length of the slug were both measured. The mean liquid superficial flow velocity, v and apparent viscosity of the fluid, $\eta$ were varied by repeating experiments in the presence of 0, 10, 20, 30, 40 and 50 v/v% of glycerol. The data in Fig. 22 shows the deposited film thickness follows closely the Taylor's law, in which the reduction in length is proportional to the capillary number, given by: $Ca = \eta^*v/\gamma$, where $\gamma$ is the surface tension of glycerol:water mixtures.

Surprisingly, the amount of fluid deposited increases linearly with the mean superficial flow velocity. Such a result is surprising when convective forces are considered alone. It should be noted that shear increases with fluid velocity, which is believed to make the film thinner.

Full deposition of the fluid slug is possible by using longer lengths of microcapillaries or shorter lengths of slugs. The full volume of a 10 mm long water slug was 100% deposited in a 30cm long capillary using a superficial flow velocity of 30 mm/s. The deposited thin liquid film can be dried by passing an inert gas like nitrogen through the microcapillary.

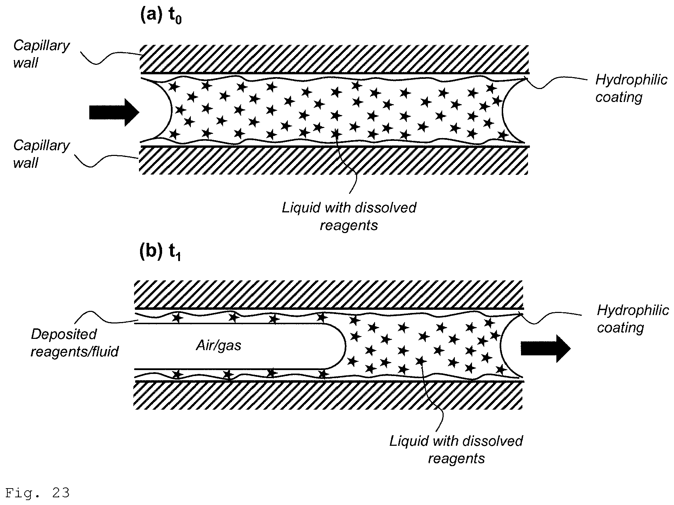

An alternative method of deposition consists in fully loading the microcapillaries with the assay reagent dissolved in a fluid, such as water (Fig. 23A). The fluid is then pushed out of the microcapillary with pressurised air or inert gas, such as nitrogen. The film thickness is controlled by the capillary number, which depends on viscosity of the fluid, surface tension and mean superficial flow velocity of the air/gas during the removal of excess reagent (Fig. 23B). The deposited thin liquid film can be dried by passing an inert gas, such as nitrogen, through the microcapillary. In addition, the removed excess assay reagent can be reutilised.

An experiment was conducted to test a combination of sugar fermentation and enzymatic assays for the specific identification of bacteria. Individual 200 micron internal diameter capillaries coated with cross-linked HMW PVA were individually loaded with different reagents to perform a combination of colourimetric sugar fermentation and enzymatic assays as shown in Table 4.

**Table 4.** Reagents loaded into individual capillaries

| Loaded reagents | | |
| --- | --- | --- |
| Capillary number | Fermentation assays | Enzymatic assays |
| 1 | None | None |
| 2 | Glucose | None |
| 3 | Maltose | 4-Nitrophenyl phosphate |
| 4 | Mannitol | 4-Nitrophenyl phosphate |
| 5 | Mannose | 4-Nitrophenyl β-D-glucuronide |
| 6 | Sucrose | 4-Nitrophenyl β-D-glucuronide |
| 7 | Trehalose | 4-Nitrophenyl β-D-glucopyranoside |
| 8 | N-Acetyl Glucosamine | 4-Nitrophenyl β-D-glucopyranoside |
| 9 | Arginine | 4-Nitrophenyl β-D-galacto-pyranoside |
| 10 | Urea | 4-Nitrophenyl β-D-galacto-pyran-oside |

The sugar fermentation strips had the capillaries individually loaded with sugars at a concentration of 750 mg/mL with the exception of mannose (450 mg/ml), or arginine (700 mg/mL) containing glucose (10mg/ml) and pyridoxal (0.1 mg/mL) or a solution of urea at 500 mg/mL; the enzymatic assay strips were loaded with enzymatic substrates at concentration of 500 mg/mL (as summarised in Table 4).

The strips were then trimmed to produce short 30mm long strips, that were dipped into samples containing bacteria, either Staphylococcus Aureus ATCC 25923; Staphylococcus Epidermidis ATCC 12228 or Staphylococcus Saprophyticus ATCC 15305 resuspended in Microbatch™ Staph 12S suspending media. The strips were incubated overnight at 37°C, and positive/negative identification of assays on individual capillaries was performed by imaging the strips with CCD camera.

Sugar Fermentation: if a sugar is fermented, bacteria will produce acid end products and the pH of the medium will drop. The media contains a pH indicator to indicate acid production. A positive test for sugar fermentation consists of a colour change from orange to yellow, indicating a pH change to acidic.

Arginine and Urea degradation (detection of Arginine dihydrolase/Urease): when arginine or urea are present, the pH of the medium rises. Accordingly, a positive test for detection of arginine dihydrolase or urease consists of a colour change from orange to purple indicating a pH change to alkaline.

Enzyme detection: enzymatic substrates are normally colourless. If bacteria have the targeted enzymes, the substrates will be hydrolysed, releasing compounds that will produce a yellow colour indicating a positive reaction.

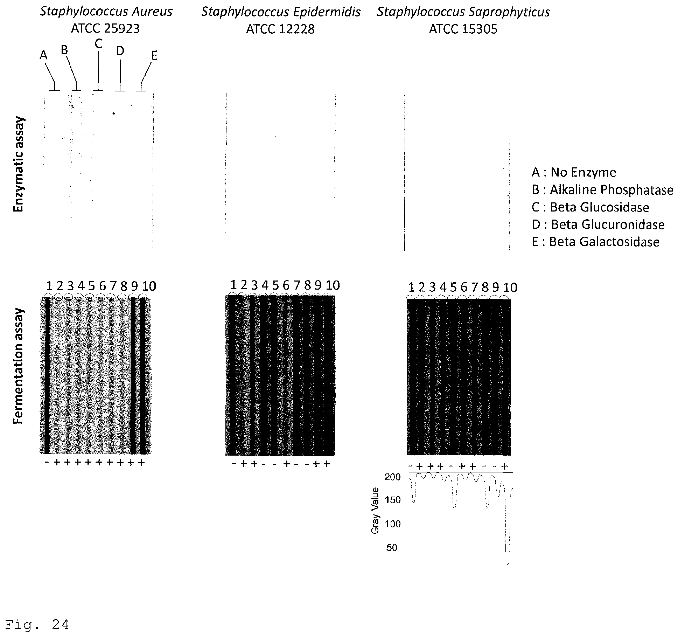

The imaged strips are shown in Fig. 24 and the bacteria identification resulting from data interpretation is summarised in Table 5.

**Table 5.** Results from colourimetric fermentation and enzymatic assays. O - orange; Y - Yellow; P - purple; T - transparent

| | | Capillary number (from left to right) | | | | | | | | | |
|---|---|---|---|---|---|---|---|---|---|---|---|
| | | **1** | **2** | **3** | **4** | **5** | **6** | **7** | **8** | **9** | **10** |
| **Fermentation assays** | *Staphylococcus Aureus* ATCC 25923 | O | Y | Y | Y | Y | Y | Y | Y | O/P | P |
| | *Staphylococcus Epidermidis* ATCC 12228 | O | Y | Y | O | O | Y | O | O | P | P |
| | *Staphylococcus Saprophyticus* ATCC 15305 | O | Y | Y | Y | O | Y | Y | O | O | P |
| **Enzymatic assays** | *Staphylococcus Aureus* ATCC 25923 | T | T | Y | Y | Y | Y | T | T | T | T |
| | *Staphylococcus Epidermidis* ATCC 12228 | T | T | T | T | Y | Y | T | T | T | T |
| | *Staphylococcus Saprophyticus* ATCC 15305 | T | T | T | T | T | T | T | T | Y | Y |

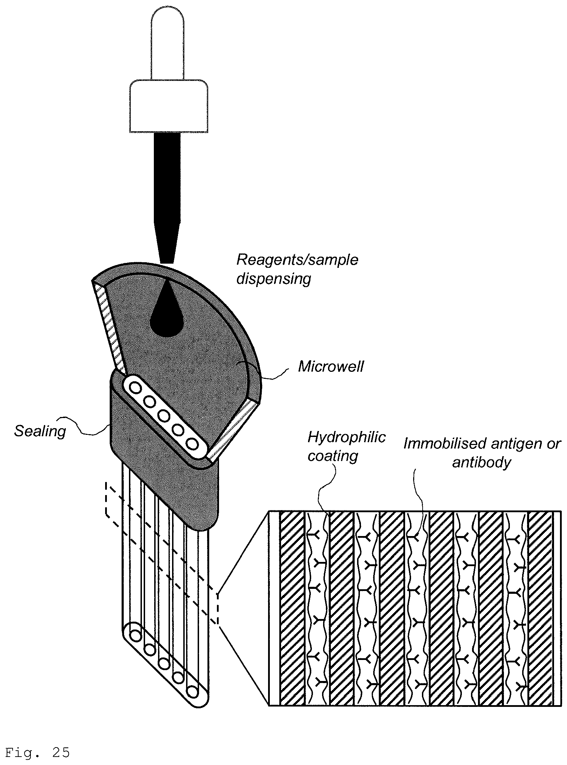

In another embodiment, a device is shown in Fig. 25 for performing immunoassays consisting of multiple capillaries coated with the polymer layer and in which antigens or antibodies are immobilised covalently in the layer. The assay can be performed based on gravity, without the need of any hydraulics. The sample and reagents can be loaded manually using a dropper, pipette, syringe or other manually liquid handling device, or automatically using e.g. a syringe pump. Such a device provides a similar functionality to the sandwich PSA assay described above (and illustrated in Fig. 20) with an alternative loading method.

REFERENCES

All references referred to herein are incorporated by reference in their entirety.

Beck, M.; Brockhuis, S.; van der Velde, N.; Breukers, C.; Greve, J.; Terstappen, L. W. M. M. Lab on a chip 2012, 12, 167-173.

Carneiro-Da-Cunha, M. G., Cerqueira, M. A., Souza, B. W. S., Carvalho, S., Quintas, M. A. C., Teixeira, J. A. & Vicente, A. A. 2010. Physical and thermal properties of a chitosan/alginate nanolayered PET film. Carbohydrate Polymers, 82, 153-159.

Chen, W, Surface Modification of solid phase objects by poly(vinyl alcohol), Patent US 7,179,506 B2, 20 February 2007.

Chin et al., Microfluidics-based diagnostics of infectious diseases in the developing world, Nat. Med., (2011) 17:1015-1019

Edwards, A. D.; Reis, N. M.; Slater, N. K. H.; Mackley, M. R. Lab on a Chip 2011, 11, 4267-4273.

Fujii, Y.; Henares, T. G.; Kawamura, K.; Endo, T.; Hisamoto, H. 2012, *12,* 1522-1526.

Gervais and Delamarche, Toward one-step point-of-care immunodiagnostics using capillary-driven microfluidics and PDMS substrates, Lab Chip, (2009) 9:3330-3337

Hitzbleck M., Gervais L., Delamarche E., Controlled release of reagents in capillary-driven microfluidics using reagent Integrators, Lab Chip, (2011) 11:2680-2685

Huang, X., Foss Jr, F. W. & Dasgupta, P. K. 2011. Multilayer chitosan-based open tubular capillary anion exchange column with integrated monolithic capillary suppressor. Analytica Chimica Acta, 707, 210-217

Kawabata et al., Electrokinetic analyte transport assay for alpha-fetoprotein immunoassay integrates mixing, reaction and separation on-chip, Electrophoresis, (2008) 7:1399-406

Kozlov, M.; Quarmyne, M.; Chen, W.; McCarthy, T. J. 2003. Adsorption of Poly (vinyl alcohol) onto Hydrophobic Substrates . A General Approach for Hydrophilizing and Chemically Activating Surfaces. Macromolecules 2003, 36, 6054-6059.

Lee et al., A fully automated immunoassay from whole blood on a disc, Lab Chip, (2009) 9:1548-1555

Lin, C.-H., Lin, J.-C., Chen, C.-Y., Cheng, C.-Y., Lin, X.-Z. & Wu, J.-J. 2005. Feasibility evaluation of chitosan coatings on polyethylene tubing for biliary stent applications. Journal of Applied Polymer Science, 97, 893-302

Madou, M. J.; Lee, L. J.; Daunert, S.; Lai, S.; Shih, C. Biomedical Microdevices 2001, 3, 245-254.

Mastichiadis et al., Simultaneous Determination of Pesticides Using a Four-Band Disposable Optical Capillary Immunosensor,Anal. Chem., (2002) 74:6064-6072

Mccreath, G. E., Owen, R. O., Nash, D. C. & Chase, H. A. 1997. Preparation and use of ion-exchange chromatographic supports based on perfluoropolymers. Journal of Chromatography A, 773(1-2), 73-83.

Mohidus et al., Paper Diagnostics for Instantaneous Blood Typing, Anal. Chem., (2010) 4158-4164.

Sin et al., System Integration - A Major Step toward Lab on a Chip, Journal of Biological Engineering, (2011) 5:6

Uchiyama, Y.; Okubo, F.; Akai, K.; Fujii, Y.; Henares, T. G.; Kawamura, K.; Yao, T. Lab on a chip 2012, 12, 204-208.

van Oordt, T.; Barb, Y.; Smetana, J.; Zengerle, R.; von Stetten, F. Lab on a chip 2013, 13, 2888-2892.

Wakayama, H.; Henares, T. G.; Jigawa, K.; Funano, S.; Sueyoshi, K.; Endo, T.; Hisamoto, H. 2013, *13,* 4304-4307.

Yacoub-George et al., Automated 10-channel capillary chip immunodetector for biological agents detection, Biosens. Bioelectron., (2007) 22:1368-1375

## Claims

1. An assay device having:

   a unitary body with an exterior surface, the unitary body being substantially transparent to visible light and formed from a material having a refractive index in the range 1.26 to 1.40, the refractive index being measured at 20 °C with light of wavelength 589 nm, and wherein the unitary body is formed from a hydrophobic material, and at least two capillary bores extending internally along the unitary body, wherein at least a portion of the surface of each capillary bore includes a hydrophilic layer for retaining an assay reagent, and
   wherein the hydrophilic layer is also substantially transparent to visible light to allow optical interrogation of the capillary bores through the capillary wall.

2. An assay device according to claim 1, wherein the hydrophilic layer is coated onto the surface of one or more capillary bores.

3. An assay device according to claim 1 or claim 2 wherein an assay reagent is retained at least at a portion of the hydrophilic layer or hydrophilic layer-coated surface of one or more capillary bores,

and optionally one or more assay reagents are reversibly retained at the hydrophilic layer,
and/or optionally one or more assay reagents are irreversibly retained at the hydrophilic layer.

4. An assay device according to claim 3 wherein one capillary bore has a different assay reagent retained at the hydrophilic layer or hydrophilic layer-coated surface of the capillary bore compared with at least one other capillary bore.

5. An assay device according to claim 3 or claim 4 wherein two or more capillary bores have the same assay reagent retained at the hydrophilic layer or hydrophilic layer-coated surface of the capillary bore, in order to provide measurement redundancy in the device.

6. An assay device according to any one of claims 3 to 5, wherein the assay reagent is selected from the group consisting of: a population of first members of a respective binding pair, each first member being capable of specifically binding with a second member of the respective binding pair; an enzyme substrate; a dye; a pH indicator; a substance which inhibits or promotes growth of a microorganism; particle reagents; and a metabolic substrate for a microorganism,
and optionally the population of first members is a population of antibodies.

7. An assay device according to any one of the preceding claims wherein the hydrophilic layer comprises, or consists of, polyvinylalcohol (PVA), cross-linked polyvinylalcohol (PVA), gelatine, hydroxypropylmethylcellulose (HPMC), carboxymethyl cellulose (CMC), poly-lysine, collagen, PVA and dextrin, PVA and dextran, PVA and gelatine, cross-linked chitosan, or alginate and calcium.

8. An assay device according to any one of the preceding claims wherein the hydrophobic material is a fluoropolymer selected from the group consisting of fluorinated ethylene propylene (FEP), tetrafluoroethylene hexafluoropropylene vinylidene fluoride (THV), perfluoroalkoxy (PFA), polytetrafluoroethylene (PTFE), ethylene tetrafluoroethylene (ETFE), and poly(chlorotrifluoroethylene) (PCTFE).

9. An assay system, the system having at least one assay device according to any one of the preceding claims and a holder for holding the assay device,
and optionally the holder provides observation means to allow at least a part of the assay device to be observed.

10. A method of performing an assay for determining the presence of a substance in a sample fluid using a device according any one of claims 3 to 8, the method including the steps:

bringing a sample fluid into contact with the capillary bores of the device;

(i) allowing the sample fluid to rise in the capillary bores by capillary action thereby bringing the sample fluid into contact with the assay reagent(s) retained at the hydrophilic layer or hydrophilic layer-coated surface of the capillary bore(s); or
(ii) allowing the sample fluid to fill the capillary bores by gravity thereby bringing the sample fluid into contact with the assay reagent(s) retained at the hydrophilic layer or hydrophobic layer-coated surface of the capillary bore(s); and

optically interrogating the capillary bores through the capillary wall to determine the presence of the substance in the sample fluid,
and optionally the optical interrogation step is carried out using a digital camera, digital microscope, flatbed scanner.

11. A method for manufacturing an assay device according to any one of claims 1 to 8, the method including:

providing an extruded body having at least two capillary bores extending internally along the body, the body being substantially transparent to visible light and formed from a material having a refractive index in the range 1.26 to 1.40, the refractive index being measured at 20 °C with light of wavelength 589 nm; inserting a coating fluid into the capillary bores to coat at least a portion of the surface of each capillary bore with a hydrophilic layer for retaining assay reagents, wherein the hydrophilic layer is also substantially transparent to visible light; and
forming a coated extruded body.

**12.** A method according to claim 11 further comprising the step of

inserting a respective loading fluid into one or more capillary bores of the coated extruded body, each loading fluid comprising an assay reagent, to retain the assay reagent(s) at least at a portion of the hydrophilic layer-coated surface of the capillary bore(s), and
forming a coated and loaded extruded body,
and optionally the step of inserting a respective loading fluid into one or more capillary bores of the coated extruded body includes filling the capillary bore with loading fluid so that (i) the capillary bore is substantially filled along its length with loading fluid before excess loading fluid is removed from the capillary bore; or (ii) only part of the capillary bore is filled with loading fluid before excess loading fluid is removed from the capillary bore.

**13.** A method according to claim 11 or claim 12 wherein the assay reagent is selected from the group consisting of: a population of first members of a respective binding pair, each first member being capable of specifically binding with a second member of the respective binding pair; an enzyme; an enzyme substrate; a dye; a pH indicator; a substance which inhibits or promotes growth of a microorganism; particle reagents; and a metabolic substrate for a microorganism,
and optionally the population of first members is a population of antibodies.

**14.** A method according to any one of claims 11 to 13 further including the step of cutting the coated, or coated and loaded, extruded body to form an assay device of a required length, wherein the coated, or coated and loaded, extruded body, before cutting, optionally has a length of at least 20 cm.

**15.** A method according to any one of claims 11 to 13, being a method for manufacturing a set of n assay devices, the method further including cutting the coated, or coated and loaded, extruded body to form the set of n devices, each device having a length of at least X,
wherein the coated, or coated and loaded, extruded body, before cutting, has a length of at least nX, or a length of at least 20 cm.

**Patentansprüche**

**1.** Testvorrichtung, die Folgendes umfasst:

einen einheitlichen Körper mit einer Außenoberfläche, wobei der einheitliche Körper für sichtbares Licht im Wesentlichen durchlässig ist und aus einem Material besteht, das einen Brechungsindex im Bereich von 1,26 bis 1,40 aufweist, wobei der Brechungsindex bei 20 °C mit Licht mit einer Wellenlänge von 589 nm gemessen wird, und wobei der einheitliche Körper aus einem hydrophoben Material besteht, sowie
zumindest zwei Kapillarbohrungen, die sich im Inneren entlang des einheitlichen Körpers erstrecken, wobei zumindest einen Teil der Oberfläche jeder Kapillarbohrung eine hydrophile Schicht zum Zurückhalten eines Testreagens umfasst, und
wobei die hydrophile Schicht ebenfalls für sichtbares Licht im Wesentlichen durchlässig ist, um eine optische Abfrage der Kapillarbohrungen durch die Kapillarwand hindurch zu ermöglichen.

**2.** Testvorrichtung nach Anspruch 1, wobei die Oberfläche einer oder mehrerer Kapillarbohrungen mit der hydrophilen Schicht beschichtet ist.

**3.** Testvorrichtung nach Anspruch 1 oder Anspruch 2, wobei ein Testreagens zumindest an einem Teil der hydrophilen Schicht oder der mit der hydrophilen Schicht beschichteten Oberfläche einer oder mehrerer Kapillarbohrungen zurückgehalten wird

und gegebenenfalls ein oder mehrere Testreagenzien reversibel an der hydrophilen Schicht zurückgehalten werden
und/oder gegebenenfalls ein oder mehrere Testreagenzien irreversibel an der hydrophilen Schicht zurückgehalten werden.

**4.** Testvorrichtung nach Anspruch 3, wobei eine Kapillarbohrung ein anderes Testreagenz aufweist, das an der hydrophilen Schicht oder der mit der hydrophilen Schicht beschichteten Oberfläche der Kapillarbohrung zurückgehalten wird, als zumindest eine andere Kapillarbohrung.

5. Testvorrichtung nach Anspruch 3 oder Anspruch 4, wobei zwei oder mehr Kapillarbohrungen dasselbe Testreagenz aufweisen, das an der hydrophilen Schicht oder der mit der hydrophilen Schicht beschichteten Oberfläche der Kapillarbohrung zurückgehalten wird, um für Messredundanz in der Vorrichtung zu sorgen.

6. Testvorrichtung nach einem der Ansprüche 3 bis 5, wobei das Testreagens aus der aus folgenden bestehenden Gruppe ausgewählt ist: einer Population von ersten Elementen eines entsprechenden Bindungspaars, wobei jedes erste Element dazu in der Lage ist, spezifisch an ein zweites Element des entsprechenden Bindungspaars zu binden; einem Enzymsubstrat; einem Farbstoff; einem pH-Indikator; einer Substanz, die das Wachstum eines Mikroorganismus inhibiert oder fördert; Teilchenreagenzien; und einem Stoffwechselsubstrat für einen Mikroorganismus; und wobei gegebenenfalls die Population von ersten Elementen eine Population von Antikörpern ist.

7. Testvorrichtung nach einem der vorangegangenen Ansprüche, wobei die hydrophile Schicht Polyvinylalkohol (PVA), vernetzten Polyvinylalkohol (PVA), Gelatine, Hydroxypropylmethylcellulose (HPMC), Carboxymethylcellulose (CMC), Polylysin, Collagen, PVA und Dextrin, PVA und Dextran, PVA und Gelatine, vernetztes Chitosan oder Alginat und Calcium umfasst oder daraus besteht.

8. Testvorrichtung nach einem der vorangegangenen Ansprüche, wobei das hydrophobe Material ein Fluorpolymer ist, das aus der aus fluoriertem Ethylen-Propylen (FEP), Tetrafluorethylen-Hexafluorpropylen-Vinylidenfluorid (THV), Perfluoralkoxy (PFA), Polytetrafluorethylen (PTFE), Ethylen-Tetrafluorethylen (ETFE) und Poly(chlortrifluorethylen) (PCTFE) bestehenden Gruppe ausgewählt ist.

9. Testsystem, wobei das Testsystem zumindest eine Testvorrichtung nach einem der vorangegangenen Ansprüche und eine Halterung zum Halten der Testvorrichtung, wobei gegebenenfalls die Halterung Beobachtungsmittel bereitstellt, um zumindest einen Teil der Testvorrichtung beobachten zu können.

10. Verfahren zum Durchführen eines Tests zum Nachweisen der Gegenwart einer Substanz in einem Probenfluid unter Verwendung einer Vorrichtung nach einem der Ansprüche 3 bis 8, wobei das Verfahren die folgenden Schritte umfasst:

    das In-Kontakt-Bringen eines Probenfluids mit den Kapillarbohrungen der Vorrichtung;

    (i) das Aufsteigenlassen des Probenfluids in den Kapillarbohrungen aufgrund von Kapillarwirkung, wodurch das Probenfluid mit dem/den Testreagens/Testreagenzien in Kontakt gebracht wird, das/die an der hydrophilen Schicht oder der mit der hydrophilen Schicht bedeckten Oberfläche der Kapillarbohrung(en) zurückgehalten wird/werden; oder
    (ii) das Füllenlassen der Kapillarbohrungen mit dem Probenfluid aufgrund der Schwerkraft, wodurch das Probenfluid mit dem/den Testreagens/Testreagenzien in Kontakt gebracht wird, das/die an der hydrophilen Schicht oder der mit der hydrophilen Schicht bedeckten Oberfläche der Kapillarbohrung(en) zurückgehalten wird/werden; und

    gegebenenfalls das Abfragen der Kapillarbohrungen durch die Kapillarwand hindurch, um die Gegenwart der Substanz in dem Probenfluid zu bestimmen,
    und wobei der Abfrageschritt gegebenenfalls unter Verwendung einer Digitalkamera, einem Digitalmikroskop oder einem Flachbettscanner durchgeführt wird.

11. Verfahren zur Herstellung einer Testvorrichtung nach einem der Ansprüche 1 bis 8, wobei das Verfahren Folgendes umfasst:

    das Bereitstellen eines extrudierten Körpers mit zumindest zwei Kapillarbohrungen, die sich im Inneren entlang des Körpers erstrecken, wobei der Körper für sichtbares Licht im Wesentlichen durchlässig ist und aus einem Material besteht, das einen Brechungsindex im Bereich von 1,26 bis 1,40 aufweist, wobei der Brechungsindex bei 20 °C mit Licht einer Wellenlänge von 589 nm gemessen wird; das Einbringen eines Beschichtungsfluids in die Kapillarbohrungen, um zumindest einen Teil der Oberfläche jeder Kapillarbohrung mit einer hydrophilen Schicht zum Zurückhalten von Testreagenzien zu beschichten, wobei die hydrophile Schicht ebenfalls für sichtbares Licht im Wesentlichen durchlässig ist; und
    das Bilden eines beschichteten extrudierten Körpers.

**12.** Verfahren nach Anspruch 11, das weiters den folgenden Schritt umfasst:

das Einbringen eines entsprechenden Ladefluids in eine oder mehrere Kapillarbohrungen des beschichteten extrudierten Körpers, wobei jedes Ladefluid ein Testreagenz umfasst, um das/die Testreagens/Testreagenzien zumindest in einem Teil der mit der hydrophilen Schicht beschichteten Oberfläche der Kapillarbohrung(en) zurückzuhalten, und

das Bilden eines beschichteten und beladenen extrudierten Körpers,

sowie gegebenenfalls den Schritt des Einbringens eines entsprechenden Ladefluids in eine oder mehrere Kapillarbohrungen des beschichteten extrudierten Körpers, das die Kapillarbohrung mit Ladefluid füllt, so dass

(i) die Kapillarbohrung entlang ihrer Länge mit Ladefluid im Wesentlichen befüllt wird, bevor überschüssiges Ladefluid aus der Kapillarbohrung entfernt wird; oder

(ii) nur ein Teil der Kapillarbohrung mit dem Ladefluid befüllt wird, bevor überschüssiges Ladefluid aus der Kapillarbohrung entfernt wird.

**13.** Verfahren nach Anspruch 11 oder Anspruch 12, wobei das Testreagens aus der aus folgenden bestehenden Gruppe ausgewählt ist: einer Population aus ersten Elementen eines entsprechenden Bindungspaars, wobei das erste Element zur spezifischen Bindung an ein zweites Element des entsprechenden Bindungspaars zu binden; einem Enzym; einem Enzymsubstrat; einem Farbstoff; einem pH-Indikator; einer Substanz, die das Wachstum eines Mikroorganismus inhibiert oder fördert; einem Teilchenreagens; und einem Stoffwechselsubstrat für einen Mikroorganismus;

und wobei gegebenenfalls die Population aus ersten Elementen eine Population aus Antikörpern ist.

**14.** Verfahren nach einem der Ansprüche 11 bis 13, das weiters den Schritt des Schneidens des beschichteten oder beschichteten und beladenen extrudierten Körpers zum Bilden einer Testvorrichtung einer erforderlichen Länge umfasst, wobei der beschichtete oder beschichtete und beladene extrudierte Körper vor dem Schneiden gegebenenfalls eine Länge von zumindest 20 cm aufweist.

**15.** Verfahren nach einem der Ansprüche 11 bis 13, das ein Verfahren zur Herstellung eines Satzes aus n Testvorrichtungen ist, wobei das Verfahren weiters das Schneiden des beschichteten oder beschichteten und beladenen extrudierten Körpers zum Bilden des Satzes aus n Vorrichtungen umfasst, wobei jede Vorrichtung eine Länge von zumindest X aufweist,

wobei der beschichtete oder der beschichtete und beladene extrudierte Körper vor dem Schneiden eine Länge von zumindest nX oder eine Länge von zumindest 20 cm aufweist.

**Revendications**

**1.** Dispositif d'essai ayant :

un corps unitaire ayant une surface extérieure, le corps unitaire étant sensiblement transparent vis-à-vis d'une lumière visible et formé d'un matériau ayant un indice de réfraction dans la plage comprise entre 1,26 et 1,40, l'indice de réfraction étant mesuré à 20°C avec une lumière ayant une longueur d'onde de 589 nm, et dans lequel le corps unitaire est formé d'un matériau hydrophobe, et

au moins deux trous capillaires s'étendant intérieurement le long du corps unitaire, au moins une partie de la surface de chaque trou capillaire comprenant une couche hydrophile pour retenir un réactif d'essai, et

dans lequel la couche hydrophile est également sensiblement transparente vis-à-vis d'une lumière visible pour permettre une interrogation optique des trous capillaires à travers la paroi capillaire.

**2.** Dispositif d'analyse selon la revendication 1, dans lequel la couche hydrophile est revêtue sur la surface d'un ou plusieurs trous capillaires.

**3.** Dispositif d'essai selon la revendication 1 ou la revendication 2, dans lequel un réactif d'essai est retenu au moins au niveau d'une partie de la couche hydrophile ou de la surface revêtue d'une couche hydrophile d'un ou plusieurs trous capillaires,

et de manière facultative un ou plusieurs réactifs d'essai sont retenus de manière réversible au niveau de la couche hydrophile,

et/ou de manière facultative un ou plusieurs réactifs d'essai sont retenus de manière irréversible au niveau de la couche hydrophile.

4. Dispositif d'essai selon la revendication 3, dans lequel un trou capillaire a un réactif d'essai différent retenu au niveau de la couche hydrophile ou de la surface revêtue d'une couche hydrophile du trou capillaire par comparaison à au moins un autre trou capillaire.

5. Dispositif d'essai selon la revendication 3 ou 4, dans lequel deux ou plus de deux trous capillaires ont le même réactif d'essai retenu au niveau de la couche hydrophile ou de la surface revêtue d'une couche hydrophile du trou capillaire, afin de fournir une redondance de mesure dans le dispositif.

6. Dispositif d'essai selon l'une quelconque des revendications 3 à 5, dans lequel le réactif d'essai est choisi parmi le groupe comprenant : une population de premiers éléments d'une paire de liaison respective, chaque premier élément étant capable de se lier de manière spécifique à un second élément de la paire de liaison respective ; un substrat enzymatique ; un colorant ; un indicateur de pH ; une substance qui inhibe ou favorise la croissance d'un microorganisme ; des réactifs particulaires ; et un substrat métabolique pour un microorganisme, et de manière facultative la population de premiers éléments est une population d'anticorps.

7. Dispositif d'essai selon l'une quelconque des revendications précédentes, dans lequel la couche hydrophile comprend, ou est constituée de, un alcool polyvinylique (PVA), un alcool polyvinylique (PVA) réticulé, de la gélatine, de l'hydroxypropylméthylcellulose (HPMC), de la carboxyméthylcellulose (CMC), de la poly-lysine, du collagène, de PVA et dextrine, de PVA et dextrane, de PVA et gélatine, de chitosan réticulé, ou d'alginate et de calcium.

8. Dispositif d'essai selon l'une quelconque des revendications précédentes, dans lequel le matériau hydrophobe est un fluoropolymère choisi parmi le groupe constitué d''éthylène propylène fluoré (FEP), de fluorure de tétrafluoroéthylène hexafluoropropylène vinylidène (THV), de perfluoroalkoxy (PFA), de polytétrafluoroéthylène (PTFE), d'éthylène tétrafluoroéthylène (ETFE), et de poly(chlorotrifluoroéthylène) (PCTFE).

9. Système d'essai, le système comportant au moins un dispositif d'essai selon l'une quelconque des revendications précédentes et un support pour supporter le dispositif d'essai, et de manière facultative, le support fournit des moyens d'observation pour permettre d'observer au moins une partie du dispositif d'essai.

10. Procédé pour effectuer un essai afin de déterminer la présence d'une substance dans un fluide échantillon en utilisant un dispositif selon l'une quelconque des revendications 3 à 8, le procédé comprenant les étapes consistant à :

amener un fluide échantillon en contact avec les trous capillaires du dispositif ;

(i) permettre au fluide échantillon de monter dans les trous capillaires par action capillaire, amenant ainsi le fluide échantillon en contact avec le ou les réactifs d'essai retenus au niveau de la couche hydrophile ou de la surface revêtue d'une couche hydrophile du ou des trous capillaires ; ou
(ii) permettre au fluide échantillon de remplir les trous capillaires par gravité, amenant ainsi le fluide échantillon en contact avec le ou les réactifs d'essai retenus au niveau de la couche hydrophile ou de la surface revêtue d'une couche hydrophobe du ou des trous capillaires ; et

interroger optiquement les trous capillaires à travers la paroi capillaire pour déterminer la présence de la substance dans le fluide échantillon, et de manière facultative l'étape d'interrogation optique est réalisée à l'aide d'un dispositif de prise de vue numérique, d'un microscope numérique, d'un scanner à plat.

11. Procédé de fabrication d'un dispositif d'essai selon l'une quelconque des revendications 1 à 8, le procédé comprenant de :

fournir un corps extrudé ayant au moins deux trous capillaires s'étendant intérieurement le long du corps, le corps étant sensiblement transparent vis-à-vis d'une lumière visible et formé d'un matériau ayant un indice de réfraction dans la plage comprise entre 1,26 et 1,40, l'indice de réfraction étant mesuré à 20°C avec une lumière ayant une longueur d'onde de 589 nm ; insérer un fluide de revêtement dans les trous capillaires pour revêtir au moins une partie de la surface de chaque trou capillaire d'une couche hydrophile pour retenir des réactifs d'essai, dans lequel la couche hydrophile est également sensiblement transparente vis-à-vis d'une lumière visible ; et
former un corps extrudé revêtu.

**12.** Procédé selon la revendication 11, comprenant en outre l'étape consistant à

insérer un fluide de chargement respectif dans un ou plusieurs trous capillaires du corps extrudé revêtu, chaque fluide de chargement comprenant un réactif d'essai, pour retenir le ou les réactifs d'essai au moins au niveau d'une partie de la surface revêtue d'une couche hydrophile du ou des trous capillaires, et
former un corps extrudé revêtu et chargé,
et de manière facultative, l'étape d'insertion d'un fluide de chargement respectif dans un ou plusieurs trous capillaires du corps extrudé revêtu comprend le remplissage du trou capillaire avec un fluide de chargement de sorte que (i) le trou capillaire est sensiblement rempli le long de sa longueur avec du fluide de chargement avant qu'un fluide de chargement en excès soit retiré du trou capillaire ; ou (ii) seule une partie du trou capillaire est remplie de fluide de chargement avant qu'un fluide de chargement en excès ne soit retiré du trou capillaire.

**13.** Procédé selon la revendication 11 ou la revendication 12, dans lequel le réactif d'essai est choisi parmi le groupe comprenant : une population de premiers éléments d'une paire de liaison respective, chaque premier élément étant capable de se lier spécifiquement à un second élément de la paire de liaison respective ; une enzyme ; un substrat enzymatique ; un colorant ; un indicateur de pH ; une substance qui inhibe ou favorise la croissance d'un microorganisme ; des réactifs particuliers ; et un substrat métabolique pour un microorganisme,
et de manière facultative la population de premiers éléments est une population d'anticorps.

**14.** Procédé selon l'une quelconque des revendications 11 à 13, comprenant en outre l'étape consistant à couper le corps extrudé revêtu, ou revêtu et chargé, pour former un dispositif d'essai d'une longueur requise, le corps extrudé revêtu, ou revêtu et chargé, avant la découpe, a de manière facultative une longueur d'au moins 20 cm.

**15.** Procédé selon l'une quelconque des revendications 11 à 13, qui est un procédé de fabrication d'un ensemble de n dispositifs d'essai, le procédé consistant en outre à couper le corps extrudé revêtu, ou revêtu et chargé, pour former l'ensemble de n dispositifs, chaque dispositif ayant une longueur d'au moins X,
dans lequel le corps extrudé revêtu, ou revêtu et chargé, avant la découpe, a une longueur d'au moins nX, ou une longueur d'au moins 20 cm.

Fig. 1

Fig. 2

Fig. 3

Fig. 4D

Fig. 4C

Fig. 4B

Fig. 4A

FEP:
RI=1.34

FEP:
RI=1.34

Air: RI = 1.00

Water:
RI=1.33

Fused silica,
RI=1.46

Fig. 5A

Fig. 5B

Hydrophilic polymer coating

Sample drawn into capillary

Reagent loaded in capillary

Reagent released into sample

Drop of sample

Fig. 6

Fig. 7A

# No cross-linking

□ 1st measurement ▨ 2nd measurement ▨ 3rd measuremenet

**Fig. 7B**

# 2h cross-linking

□ 1st measurement ▨ 2nd measurement □ 3rd measurement

**Fig. 7C**

Fig. 7D

Fig. 7E

Fig. 8

Polymer B  Polymer C  Crosslinked polymer A

Fig. 9

Fig. 10

Fig. 11

(1)
Bulk immobilisation of
ABO/Rh reagents

200

202

neg. control

anti-A

anti-B

anti-D

neg. control

Fig. 12

(2)
Cut into 100 mm long
test strips

204

Fig. 13

(3)
Multiplex dip-stick

Fig. 14

porous hydrophilic layer with retained antibody reagents

capillary wall

air-liquid interface

drop of blood

**Fig. 15**

A+ blood

100 mm

anti-    anti-    anti-
A        B        D

**Fig. 16A**

**Fig. 16B**

Anti-B: negative

(constant red blood cells concentration along $x$

**Fig. 16C**

Anti-D: positive

(decrease in red blood cells concentration along $x$

**Fig. 16D**

**Fig. 17A**

Agglutinated red    Plasma/buffe    Air in
blood cells        r separated     capillary
                   from red        head

**Fig. 17B**

Profile plot for 3rd capillary

Distance (pixels)

**Fig. 17C**

**Fig. 18A**

| Blood sample | #1 | #2 | #3 |
|---|---|---|---|
| Control | 0 | 0 | 0 |
| Anti-A | + | 0 | 0 |
| Anti-B | 0 | + | 0 |
| Anti-D | + | + | + |
| Detected blood type | A⁺ | B⁺ | O⁺ |

**Fig. 18B**

Fig. 18C

Fig. 18D

Fig. 18E

Fig. 18F

Fig. 18G

Fig. 18H

Fig. 18I

Fig. 18J

Fig. 18K

Fig. 18L

Fig. 19

Fig. 20

**(a) t$_0$**

Capillary
wall

Air/gas

Hydrophilic
coating

Capillary
wall

Liquid with dissolved
reagents

**(b) t$_1$**

Deposited
reagents/fluid

Air/gas

Air/gas

Hydrophilic
coating

Liquid with dissolved
reagents

Fig. 21

Fig. 22a

Fig. 22B

**(a) t$_0$**

Capillary
wall

Hydrophilic
coating

Capillary
wall

Liquid with dissolved
reagents

**(b) t$_1$**

Hydrophilic
coating

Deposited
reagents/fluid

Air/gas

Liquid with dissolved
reagents

Fig. 23

Fig. 24

Reagents/sample
dispensing

Microwell

Sealing

Hydrophilic
coating

Immobilised antigen or
antibody

Fig. 25

Cross-section

Top view

Plastic wall

Hydrophilic
coating

Loaded reagents

Fig. 26

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011117579 A, Edwards
- WO 2011143075 A2, Beck
- US 2008020453 A
- US 2002094533 A
- WO 03042697 A
- US 7179506 B2
- WO 2005056272 A

### Non-patent literature cited in the description

- **BECK, M. ; BROCKHUIS, S. ; VAN DER VELDE, N. ; BREUKERS, C. ; GREVE, J. ; TERSTAPPEN, L. W. M. M.** *Lab on a chip,* 2012, vol. 12, 167-173
- **CARNEIRO-DA-CUNHA, M. G. ; CERQUEIRA, M. A. ; SOUZA, B. W. S. ; CARVALHO, S. ; QUINTAS, M. A. C. ; TEIXEIRA, J. A. ; VICENTE, A. A.** Physical and thermal properties of a chitosan/alginate nanolayered PET film. *Carbohydrate Polymers,* 2010, vol. 82, 153-159
- **CHIN et al.** Microfluidics-based diagnostics of infectious diseases in the developing world. *Nat. Med.,* 2011, vol. 17, 1015-1019
- **EDWARDS, A. D. ; REIS, N. M. ; SLATER, N. K. H. ; MACKLEY, M. R.** *Lab on a Chip,* 2011, vol. 11, 4267-4273
- **GERVAIS ; DELAMARCHE.** Toward one-step point-of-care immunodiagnostics using capillary-driven microfluidics and PDMS substrates. *Lab Chip,* 2009, vol. 9, 3330-3337
- **HITZBLECK M. ; GERVAIS L. ; DELAMARCHE E.** Controlled release of reagents in capillary-driven microfluidics using reagent Integrators. *Lab Chip,* 2011, vol. 11, 2680-2685
- **HUANG, X. ; FOSS JR, F. W. ; DASGUPTA, P. K.** Multilayer chitosan-based open tubular capillary anion exchange column with integrated monolithic capillary suppressor. *Analytica Chimica Acta,* 2011, vol. 707, 210-217
- **KAWABATA et al.** Electrokinetic analyte transport assay for alpha-fetoprotein immunoassay integrates mixing, reaction and separation on-chip. *Electrophoresis,* 2008, vol. 7, 1399-406
- **KOZLOV, M. ; QUARMYNE, M. ; CHEN, W. ; MCCARTHY, T. J.** Adsorption of Poly (vinyl alcohol) onto Hydrophobic Substrates . A General Approach for Hydrophilizing and Chemically Activating Surfaces. *Macromolecules,* 2003, vol. 36, 6054-6059
- **LEE et al.** A fully automated immunoassay from whole blood on a disc. *Lab Chip,* 2009, vol. 9, 1548-1555
- **LIN, C.-H. ; LIN, J.-C. ; CHEN, C.-Y. ; CHENG, C.-Y. ; LIN, X.-Z. ; WU, J.-J.** Feasibility evaluation of chitosan coatings on polyethylene tubing for biliary stent applications. *Journal of Applied Polymer Science,* 2005, vol. 97, 893-302
- **MADOU, M. J. ; LEE, L. J. ; DAUNERT, S. ; LAI, S. ; SHIH, C.** *Biomedical Microdevices,* 2001, vol. 3, 245-254
- **MASTICHIADIS et al.** Simultaneous Determination of Pesticides Using a Four-Band Disposable Optical Capillary Immunosensor. *Anal. Chem.,* 2002, vol. 74, 6064-6072
- **MCCREATH, G. E. ; OWEN, R. O. ; NASH, D. C. ; CHASE, H. A.** Preparation and use of ion-exchange chromatographic supports based on perfluoropolymers. *Journal of Chromatography A,* 1997, vol. 773 (1-2), 73-83
- **MOHIDUS et al.** Paper Diagnostics for Instantaneous Blood Typing. *Anal. Chem.,* 2010, 4158-4164
- **SIN et al.** System Integration - A Major Step toward Lab on a Chip. *Journal of Biological Engineering,* 2011, vol. 5, 6
- **UCHIYAMA, Y. ; OKUBO, F. ; AKAI, K. ; FUJII, Y. ; HENARES, T. G. ; KAWAMURA, K. ; YAO, T.** *Lab on a chip,* 2012, vol. 12, 204-208
- **VAN OORDT, T. ; BARB, Y. ; SMETANA, J. ; ZENGERLE, R. ; VON STETTEN, F.** *Lab on a chip,* 2013, vol. 13, 2888-2892
- **YACOUB-GEORGE et al.** Automated 10-channel capillary chip immunodetector for biological agents detection. *Biosens. Bioelectron.,* 2007, vol. 22, 1368-1375